# EUROPEAN PATENT SPECIFICATION

(11) **EP 2 093 228 B1**
(45) Date of publication and mention of the grant of the patent: **22.02.2012**
(21) Application number: 07816580.0
(22) Date of filing: 16.10.2007
(51) Int. Cl.: C07D 495/04, C07D 487/04, A61K 31/4365, A61K 31/437, A61P 35/00, A61P 31/00, A61P 37/00, A61P 9/00, A61P 9/10, A61P 5/00

(54) **PYRROLINE-2-ONE DERIVATIVES AGAINST CELL RELEASING TUMOR NECROSIS FACTOR, PREPARATION METHODS AND USES THEREOF**
PYRROLIN-2-ON-DERIVATE GEGEN TUMORNEKROSEFAKTOR FREISETZENDE ZELLEN, HERSTELLUNGSVERFAHREN UND ANWENDUNGEN DAVON
DÉRIVÉS DE PYRROLINE-2-ONE UTILISÉS CONTRE LE FACTEUR DE NÉCROSE TUMORALE À LIBÉRATION CELLULAIRE, PROCÉDÉS DE PRÉPARATION ET UTILISATIONS DE CEUX-CI

(30) Priority: 15.11.2006 CN 200610129420
(43) Date of publication of application: 26.08.2009
(73) Proprietor: Tianjin Hemay Bio-Tech Co., Ltd., Tianjin 300457 (CN)
(72) Inventor: ZHANG, Hesheng, Tianjin 300457 (CN)
(74) Representative: Korga, Leokadia
(86) International application number: PCT/CN2007/002966
(87) International publication number: WO 2008/058449

(56) References cited:
- WO-A-98/54170
- WO-A-2007/084455
- CN-A- 1 844 118
- CN-A- 1 939 922
- US-A1- 2006 019 928
- US-A1- 2006 199 836

## Description

The invention relates to pyrroline-dione derivatives as inhibitors of tumor necrosis factor (TNF) released by cells, a method of their preparation, and a method of using the same as pharmaceutical agents.

Tumor necrosis factor-alpha (TNFα) is a cytokine, mainly produced by mononuclear macrophages. It causes inflammation, fever, cardiovascular dysfunction, hemorrhage, blood coagulation and a series of acute reactions similar to acute infection and shock when administered to humans and animals. Moreover, excessive or uncontrolled TNFα in animals or humans often indicates one of the following diseases:

1) Endotoxaemia and/or toxic shock syndrome (Tracey et al., Nature 330, 662-4 1987; Hinshaw et al., Circ Shock 30, 279-92 (1990));

2) Cachexia (Dezube et al., Laucet, 335(8690), 662(1990)); or

3) Adult Respiratory Distress Syndrome (ARDS) (Millar et al., Laucet 2(8665), 712-714(1989)).

TNFα also plays an important role in bone resorption diseases including arthritis (Betolinni et al., Nature 319, 516-8 (1986)). Furthermore, experiments in vitro and vivo have shown that TNFα may stimulate bone resorption by stimulating formation and activation of osteoclasts and inhibit the formation of bone.

At present, the disease most commonly linked to TNFα released by tumor and host tissue is hypercalcemia, which is closely related to malignant tumors (Calci. Tissue Int. (US) 46(Suppl.), S3-10(1990)). It has also been observed that immune response is closely related to an increased concentration of TNFα in serum of the patient after bone marrow transplantation (Holler et al., Blood, 75(4), 1011-1016(1990)).

Fatal hyperacute neurogenic syndrome brainstem-type malaria, which is the most dangerous type of malaria, is also linked to high blood levels of TNFα. When this type of malaria occurs, serum levels of TNFα are directly related to the disease, which often occurs during an acute attack of malaria in patients (Grau et al., N. Engl. J. Med. 320(24), 1586-91(1989)).

TNFα also plays an important role in chronic pneumonia. The storage of silicon-containing particles can cause silicosis. Silicosis is a type of progressive respiratory failure, resulting from fibrosis of pulmonary tissues. In an animal pathological model, a TNFα antibody can fully block the progress of lung fibrosis in mice caused by silica dust (Pignet et al., Nature, 344:245-7 (1990)). It was also proved that TNFα levels are abnormally high in serum of animals with pulmonary fibrosis caused by silica dust or asbestos dust in animal experiments (Bissonnette et al., Inflammation 13(3), 329-339(1989)). Pathological research reveals that TNFα levels in pulmonary tissues of patients with pulmonary sarcoidosis is much higher than that of ordinary people (Baughman et al., J. Lab. Clin. Med. 115(1), 36-42(1990)). This suggests that TNFα inhibitors may have a great significance in the treatment of chronic pulmonary diseases and lung injury.

One reason for inflammation occurring in patients with reperfusion injury may be abnormal levels of TNFα, and TNFα is regarded as the chief cause inducing tissue injury caused by ischemia (Uadder et al., PNAS 87, 2643 -6(1990)).

Besides, it has been shown that TNFα may start retroviral replication comprising that of HIV-1 (Duh et al., Proc. Nat. Acad. Sci., 86, 5974-8(1989)). T-cells need to be activated before HIV invades them. Once the activated T -cells are infected by virus (HIV), those T-cells must remain in an activated state so that the HIV virus genes are able to express and/or replicate successfully. Cytokines, especially TNFα, play an important role in the process of HIV protein expression or viral replication regulated by T-cells. Therefore, inhibition of TNFα production can in turn inhibit HIV replication in T-cells (Poll et al., Proc. Nat. Acad. Sci., 87,782-5(1990); Monto et al., Blood 79, 2670 (1990); Poll et al., AIDS Res. Human Retrovirus, 191-197(1992)).

cAMP can regulate many functions of cells, such as inflammation response, including asthma, and inflammation (Lome and Cheng, Drugs of the futune, 17(9), 799-807, 1992). When inflammation occurs, increased cAMP concentration in white cells inhibits activation of white cells, and then releases inflammation regulatory factors including TNFα so as to exacerbate inflammation. Consequently, inhibition of TNFα release can alleviate inflammation diseases including asthma.

Yu Yanyan et al. have found that TNFα plays an important role in the process of liver necrosis in patients with viral hepatitis. (Yu Yanyan etc., Chinese Journal of Internal Medicine 1996, 35:28-31). This shows that TNFα inhibitors may play a great role in treatment of chronic hepatic disease and liver injury.

Li Yingxu et al have found that levels of synthesis and secretion of tumor necrosis factors in monocytes in the peripheral blood of patients with chronic hepatic disease increase, which induces secretion of other cytokines (for example, IL-1β, IL-6 and IL-8). All these cytokines including tumor necrosis factors are all together involved in the injury process of hepatocytes (Journal of Qiqihar Medical Colleg, 22(10):1119-1120, 2001). Their study results coincide with the conclusions of Yoshioka, et al. (Hepatology, 1989, 10:769-777) and Wang Xin, et al. (Chinese Journal of Infectious Diseases, 1997, 15(2): 85-88). It has also been found that thalidomide, the inhibitor of TNFα, is able to inhibit TNFα secretion of monocytes in the peripheral blood of hepatitis patients, which lays foundation for the application of TNFα inhibitors for treatment of hepatitis, cirrhosis, and liver cancer.

By promoting biosynthesis and release of inflammatory cytokines (Abboud H.E. Kidney Int. 1993, 43: 252-267), increasing expression of cellular adhesion molecules (Egido J. et al, Kidney Int. 1993, 43(suppl 39): 59-64), and stimulating biosynthesis and release of prostaglandin G2 (PGG2) and platelet-activating factor (PAF) (Cammusi G. et al, Kidney Int., 43(suppl 39): 32 -36), TNFα may induce a series of inflammatory responses, including aggregation and adhesion of inflammatory cells, increase dilation and permeability of blood capillaries, induce fever, increase blood levels of neutrophilic granulocytes, and change hemodynamics leading to injury of renal cells. Many studies have suggested that TNFα plays an important role in breakout and deterioration of nephritis.

TNFα is involved in the regulation of immune functions by means of activation of macrophages, immunological stimulation of proliferation of T -lymphocytes, regulating the differentiation of B lymphocytes and enhancing the cytotoxicity of natural killer cells (NK).

Therefore, decreasing TNFα levels and/or increasing cAMP levels constitutes an effective way for treatment of many inflammatory, infectious, immune, or malignant tumor diseases, including but not limited to septic shock, endotoxic shock, hemodynamic shock, septic syndrome, post ischemic reperfusion injury, malaria, mycobacterial infection, meningitis, psoriasis, congestive heart failure, fibrotic disease, cachexia, transplant immune rejection, cancer, autoimmune disease, opportunistic infection in AIDS, rheumatoid arthritis (RA), hepatitis, nephritis, rheumatoid spondylitis, and so on.

In recent years, TNFα antibodies have made a breakthrough in the clinical treatment of arthritis, and have become an indispensable tool in the treatment of arthritis. However, antibody drugs have disadvantages such as high price, difficult production and immunotoxicity. Accordingly, research and development on small molecule TNFα inhibitors having low toxicity and high efficiency is of great social benefit and has high economic value.

WO98/54170A disclosed substituted 2-(2,6-dioxopiperidin-3-yl)-phthalimides and substituted 2-(2,6-dioxopiperidin-3-yl)-1-oxoisoindolines, and the method for reducing levels of TNFα and treating inflammatory and autoimmune diseases in a mammal through the administration thereof.

WO2007/084455A disclosed novel hydantoin derivatives that can inhibit matrix metalloproteinases (MMPs), a disintegrin and metalloproteases (ADAMs) and/or tumor necrosis factor α-conveting enzyme (TACE) and in so doing prevent the release of TNFα.

CN-A-1 939922 disclosed 5H-thieno(3,4-c)pyrrole-4,6-dione derivatives which are active as inhibitors of TNF released by cells.

US 2006019928 A1 disclosed tricyclic-heteroaryl compounds useful for treating p38 kinase-associated conditions.

US2006199836 A1 disclosed thienopyridine compounds capable of modulating the stability and/or activity of hypoxia inducible factor (HIF).

CN-A-1 844118 disclosed piperidine-2,6-dione derivatives which are active as inhibitors of T released by cells.

However, additional small molecule TNFα inhibitors that have low toxicity and higher efficiency are needed.

In view of the above-described problems, it is one objective of the invention to provide a compound or pharmaceutically acceptable salt or hydrate thereof which inhibits the release of TNFα in cells.

It is another objective of the invention to provide a pharmaceutical preparation comprising a compound or pharmaceutically acceptable salt or hydrate thereof which inhibits the release of TNFα in cells.

It is still another objective of the invention to provide a method of preparing a compound which inhibits the release of TNFα in cells.

To achieve the above objectives, in accordance with one embodiment of the invention, provided is a compound of Formula (**I**) or Formula (**II**),

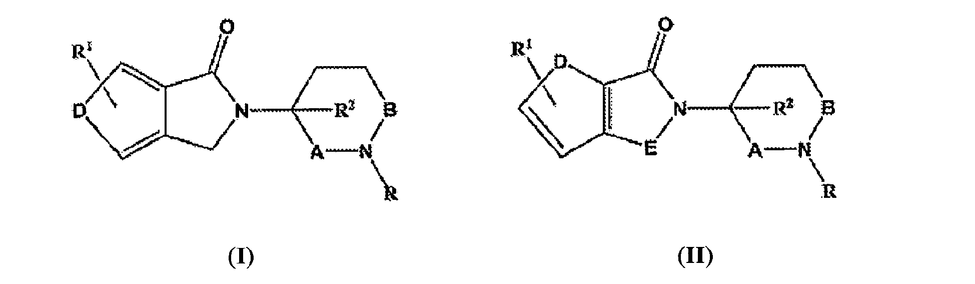

wherein

A, B and E independently represent CH₂, or CO;

D represents S, NH, or NC₁₋₆ alkylhydrocarbyl;

R represents H, C₁₋₆alkylhydrocarbyl, or R³,

R¹ at each occurrence independently represents H, or independently one or two same or different occurrences of F, Cl, Br, C_{1 -4}alkylhydrocarbyl, OH, OC₁₋₄alkylhydrocarbyl, NO₂, NHC(O)C_{1 -4}alkylhydrocarbyl, NH₂, NH(C₁₋₄alkylhydrocarbyl), or N(C_{1 -4}alkylhydrocarbyl)₂;

R² at each occurrence represents F, CF₃, H, or C₁₋₄alkyl,

R³ at each occurrence represents (CH₂)ₘR⁴, and m represents an integer from 1 to 6;

R⁴ represents F, Cl, H, C₁₋₄alkylhydrocarbyl, OH, OC, ₋₄alkylhydrocarbyl, NHC(O)C₁₋₄alkylhydrocarbyl, NH₂, NH(C_{1 -4}alkylhydrocarbyl), N(C₁₋₄alkylhydrocarbyl)₂, or O₂CR⁵;

R⁵ represents CHR⁶NR⁷R⁸, CHR⁶NR⁹C(O)CHR¹⁰NR⁷R⁸, heterocycle W, or CHR⁶NR⁹C(O)W;

R⁶, R⁹ and R¹⁰ independently represent H, or C_{1 -4}alkylhydrocarbyl;

R⁷ and R⁸ independently represent H, C₁₋₄alkylhydrocarbyl or taken together in combination represent 1,3-propylidene, 1,4-butylene, 1,5-pentylene, or 1,6-hexylidene, and

W represents saturated or unsaturated four to eight-membered heterocycle.

When W represents a heterocycle, the heterocycle is a 4 to 8 -membered saturated or unsaturated heterocycle or aromatic heterocycle and comprises one or more heteroatoms, such as N, O or S, particularly 2-pyridyl, 3-pyridyl, 4-pyridyl, 2-pyrimidinyl, 3-pyrimidinyl, 4-pyrimidinyl, or is a heterocycle selected from a compound of Formula (**III**), (**IV**), (**V**), or (**VI**), wherein G represents O, S,-NR", Y represents 1,2-ethylidene, 1,3 -propylidene, 1,4-butylene, 1,5-pentylene, 1,6-hexylidene, CH₂OCH₂, CH₂SCH₂, or CH₂NR¹²CH₂, and R¹¹, R¹² independently represent H, C_{1 -4}alkylhydrocarbyl.

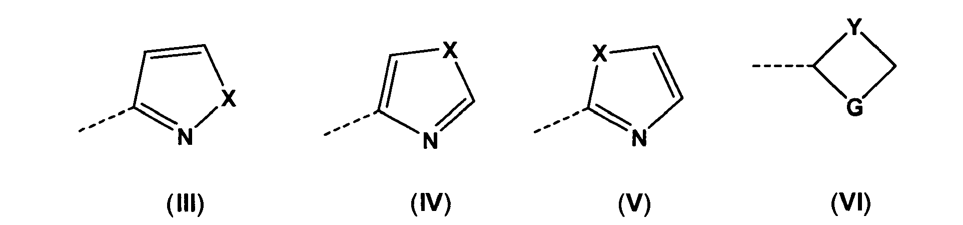

When R⁴, R⁶, R⁷, R⁸, R⁹, R¹⁰, R¹¹, and R¹² independently represent C₁ -₄alkylhydrocarbyl, C₁₋₄alkylhydrocarbyl is a straight chain or branched chain alkylhydrocarbyl, and may be substituted with F, CN, OH, COOH, C(O)NH₂, NHC(O)R¹³, NR¹⁴R¹⁵, NHC(O)NH₂, NHC(NH)NH₂, OR¹⁶, SR¹⁷, phenyl, or substituted phenyl, wherein R¹³, R¹⁴, R¹⁵, R¹⁶ and R¹⁷ independently represent H or C₁₋₄alkylhydrocarbyl.

R⁷ and R⁸ taken together in combination represent 1,3-propylidene, 1,4 -butylene, 1,5-pentylene, or 1,6-hexylidene, and may be substituted by F, CN, OH, COOH, C(O)NH₂, NHC(O)R¹³, NR¹⁴R¹⁵, NHC(O)NH₂, NHC(NH)NH₂, OR¹⁶, SR¹⁷, phenyl, or substituted phenyl, wherein R¹³, R¹⁴, R¹⁵, R¹⁶, and R¹⁷ independently represent H or C₁₋₄alkylhydrocarbyl.

In one embodiment of the invention, provided are experiments showing inhibition of TNFα in peripheral blood mononuclear cells (PBMCs) stimulated by lipopolysaccharide (LPS) by a compound represented by Formula (**I**), or Formula (**II**). Results of the experiments are listed in Table 1. The results show that the activity of most compounds of the invention is higher than that of thalidomide, a widely-used clinical pharmaceutical composition.

The compound of Formula (**I**) or Formula (**II**) suitable for being used as a pharmaceutical composition comprises the compounds wherein m represents an integer from 1 to 4, particularly 1, 2 or 3.

The compound of Formula (**I**) or Formula (**II**) suitable for being used as a pharmaceutical composition comprises the compounds wherein R¹ represents H, or one or two same or different occurrences of F, Cl, Br, CH₃, CH₂CH₃, OH, OCH₃, OCH₂CH₃, NH₂, NHCH₃, NHCH₂CH₃, N(CH₃)₂, and particularly H, F, or NH₂.

The compound of Formula (**I**) or Formula (II) suitable for being used as a pharmaceutical composition comprises the compounds wherein R² represents H, F, or CH₃.

The compound of Formula **(I)** or Formula (**II**) suitable for being used as a pharmaceutical composition comprises the compounds wherein R represents H, methyl, ethyl or (CH₂)₁₋₄R⁴, and R⁴ represents F, OH, OCH₃, OCH₂CH₃, NH₂, NHCH₃, NHCH₂CH₃, N(CH₃)₂, or O₂CR⁵, and R⁵ represents CHR⁶NR⁸R⁷, and R⁶ represents H, CH₃, CH₂CH₃, CH₂CH₂CH₃, CH(CH₃)₂, CH₂CH₂CH₂CH₃, CH₂CH(CH₃)₂, or CH(CH₃)CH₂CH₃, R⁷ and R⁸ independently represent H, CH₃, CH₂CH₃, CH₂CH₂CH₃, CH(CH₃)₂, CH₂CH₂CH₂CH₃, CH₂CH(CH₃)₂, or CH(CH₃)CH₂CH₃, or taken together in combination represent 1,4-butylene, 1,5-pentylene, or O₂CR⁵, R⁵ represents 2-pyridyl, 3-pyridyl, 4 -pyridyl, 2-pyrimidinyl, 4-pyrimidinyl, 5-pyrimidinyl, 2-pyrrolidine, 2-(*N* -methyl)pyrrolidine, 2-(*N*-ethyl)pyrrolidine, 2-(*N*-propyl)pyrrolidine, or 2-(*N* -isopropyl)pyrrolidine.

When the compound of Formula (**I**) or Formula (**II**) is an R/S isomer, it is an R isomer, or an S isomer, or a racemate.

When the compound of Formula (**I**) or Formula (**II**) is defined as an E/Z isomer, it is an E isomer, or a Z isomer, or a mixture of an E isomer and a Z isomer.

The compound of Formula (**I**) or Formula (**II**) suitable for being used as medical active ingredient may be a prodrug or a metabolite of the compound.

The compound of Formula (**I**) or Formula (**II**) suitable for being used as medical active ingredient includes but is not limited to the compounds below:

1. 3-(4-**oxo**-4*H*-thiophene[3,4-*c*]pyrrole-5(6*H*)-yl)-1-piperidine-2,6 -dione;

2. 3-(4-**oxo**-4*H*-thiophene[3,4-*c*]pyrrole-5(6*H*)-yl)-3-methylpiperidine -2,6-dione;

3. 3-(4-**oxo**-4*H*-thiophene[3,4-*c*]pyrrole-5(6*H*)-yl)-1-methylpiperidine -2,6-dione;

4. 3-(4-**oxo**-4*H*-thiophene[3,4-*c*]pyrrole-5(6*H*)-yl)-1-ethyl piperidine-2,6 -dione;

5. 3-(4-**oxo**-4*H*-thiophene[3,4*-c*]pyrrole-5(6*H*)-yl)-1-(2-methoxy -ethyl)piperidine-2,6-dione;

6. 3-(4-**oxo**-4*H*-thiophene[3,4-*c*]pyrrole-5(6*H*)-yl)-1,3-dimethyl piperidine-2,6-dione;

7. 3-(1-nitro-4-**oxo**-4*H*-thiophene[3,4-*c*]pyrrole-5(6*H*)-yl)-1-piperidine -2,6-dione;

8. 3-(1-nitro-4-**oxo**-4*H*-thiophene[3,4-*c*]pyrrole-5(6*H*)-yl)-1 -methylpiperidine-2,6-dione;

9. (±)-3-(1-nitro-4-**oxo**-4*H*-thiophene[3,4-*c*]pyrrole-5(6*H*)-yl)-3 -methylpiperidine-2,6-dione;

10. (*R*)-3-(1-nitro-4-**oxo**-4*H*-thiophene[3,4*-c*]pyrrole-5(6*H*)-yl)-3 -methylpiperidine-2,6-dione;

11. (*S*)-3-(1-nitro-4-**oxo**-4*H*-thiophene[3,4-*c*]pyrrole-5(6*H*)-yl)-3 -methylpiperidine-2,6-dione;

12. (*R*)-3-(1-amino-4-oxo-4*H*-thiophene[3, 4-*c*]pyrrole-5(6*H*)-yl)-3 -methylpiperidine-2,6-dione;

13. (*S*)-3-(1-amino-4-oxo-4*H*-thiophene[3,4*-c*]pyrrole-5(6*H*)-yl)-3 -methylpiperidine-2,6-dione;

14. (±)-3-(1-amino-4-oxo-4*H*-thiophene[3,4*-c*]pyrrole-5(6*H*)-yl)-3 -methylpiperidine-2,6-dione;

15. (*R*)-3-(1-amino-4-oxo-4*H*-thiophene[3, 4*-c*]pyrrole-5(6*H*)-yl)-1,3 -dimethyl piperidine-2,6-dione;

16. (*S*)-3-(1-amino-4-oxo-4*H*-thiophene[3,4*-c*]pyrrole-5(6*H*)-yl)-1,3 -dimethyl piperidine-2,6-dione

17. (±)-3-(1-amino-4-oxo-4*H*-thiophene[3,4-*c*]pyrrole-5(6*H*)-yl)-1,3 -dimethyl piperidine-2,6-dione;

18. (*R*)-3-(1-methylamino-4-oxo-4*H*-thiophene[3,4*-c*]pyrrole-5(6*H*)-yl)-3 -methylpiperidine-2,6-dione;

19. (*S*)-3-(1-methylamino-4-oxo-4*H*-thiophene[3,4*-c*]pyrrole-5(6*H*)-yl)-3 -methylpiperidine-2,6-dione;

20. (±)-3-(1-methylamino-4-oxo-4*H*-thiophene[3,4*-c*]pyrrole-5(6*H*)-yl)-3 -methylpiperidine-2,6-dione;

21. (*R*)-3-(1-ethylamino-4-oxo-4*H*-thiophene[3,4*-c*]pyrrole-5(6*H*)-yl)-3 -methylpiperidine-2,6-dione;

22. (*S*)-3-(1-ethylamino-4-oxo-4*H*-thiophene[3,4*-c*]pyrrole-5(6*H*)-yl)-3 -methylpiperidine-2,6-dione;

23. (±)-3-(1-ethylamino-4-oxo-4*H*-thiophene[3,4*-c*]pyrrole-5(6*H*)-yl)-3 -methylpiperidine-2,6-dione;

24. (*R*)-3-(1-dimethylamino-4-oxo-4*H*-thiophene[3,4*-c*]pyrrole-5(6*H*)-yl) -3-methylpiperidine-2,6-dione;

25. (*S*)-3-(1-dimethylamino-4-oxo-4*H*-thiophene[3,4*-c*]pyrrole-5(6*H*)-yl) -3-methylpiperidine-2,6-dione;

26. (±)-3-(1-dimethylamino-4-oxo-4*H*-thiophene[3,4*-c*]pyrrole-5(6*H*)-yl) -3-methylpiperidine-2,6-dione;

27. (*R*)-3-(1-propylamino-4-oxo-4*H*-thiophene[3,4*-c*]pyrrole-5(6*H*)-yl)-3 -methylpiperidine-2,6-dione;

28. (*S*)-3-(1-propylamino-4-oxo-4*H*-thiophene[3,4*-c*]pyrrole-5(6*H*)-yl)-3 -methylpiperidine-2,6-dione;

29. (±)-3-(1-propylamino-4-oxo-4*H*-thiophene[3,4*-c*]pyrrole-5(6*H*)-yl)-3 -methylpiperidine-2,6-dione;

30. (*R*)-3-(1-isopropylamino-4-oxo-4*H*-thiophene[3,4-*c*]pyrrole-5(6*H*)-yl) -3-methylpiperidine-2,6-dione;

31. (*S*)-3-(1-isopropylamino-4-oxo-4*H*-thiophene[3,4-*c*]pyrrole-5(6*H*)-yl) -3-methylpiperidine-2,6-dione;

32. (±)-3-(1-isopropylamino-4-oxo-4*H*-thiophene[3,4-*c*]pyrrole-5(6*H*)-yl) -3-methylpiperidine-2,6-dione;

33. (*R*)-3-(1-methylethylamino-4-oxo-4*H*-thiophene[3,4*-c*]pyrrole-5(6*H*) -yl)-3-methylpiperidine-2,6-dione;

34. (*S*)-3-(1-methylethylamino-4-oxo-4*H*-thiophene[3,4*-c*]pyrrole-5(6*H*) -yl)-3-methylpiperidine-2,6-dione;

35. (±)-3-(1-methylethylamino-4-oxo-4*H*-thiophene[3,4*-c*]pyrrole-5(6*H*) -yl)-3-methylpiperidine-2,6-dione;

36. (*R*)-3-(1-arnino-4-oxo-4*H*-thiophene[3,4-*c*]pyrrole-5(6*H*)-yl)-3-ethyl piperidine-2,6-dione;

37. (*S*)-3-(1-amino-4-oxo-4*H*-thiophene[3,4*-c*]pyrrole-5(6*H*)-yl)-3-ethyl piperidine-2,6-dione;

38. (±)-3-(1-amino-4-oxo-4*H*-thiophene[3,4*-c*]pyrrole-5(6*H*)-yl)-3-ethyl piperidine-2,6-dione;

39. (*R*)-3-(1-acetylamino-4-oxo-4*H*-thiophene[3,4-*c*]pyrrole-5(6*H*)-yl)-3 -methylpiperidine-2,6-dione;

40. (*S*)-3-(1-acetylamino-4-oxo-4*H-*thiophene[3,4*-c*]pyrrole-5(6*H*)-yl)-3 -methylpiperidine-2,6-dione;

41. (±)-3-(1-acetylamino-4-oxo-4*H*-thiophene[3,4*-c*]pyrrole-5(6*H*)-yl)-3 -methylpiperidine-2,6-dione;

42. 3-(1-amino-4-oxo-4*H*-thiophene[3,4-*c*]pyrrole-5(6*H*)-yl)-1-ethyl piperidine-2,6-dione;

43. 3-(1-amino-4-oxo-4*H*-thiophene[3,4-*c*]pyrrole-5(6*H*)-yl)-1-(2 -hydroxyethyl)piperidine-2,6-dione;

44. 3-(1-amino-4-oxo-4*H*-thiophene[3,4-*c*]pyrrole-5(6*H*)-yl)-1-propyl piperidine-2,6-dione;

45. 3-(1-amino-4-oxo-4*H*-thiophene[3,4-*c*]pyrrole-5(6*H*)-yl)-1-(3 -hydroxypropyl)piperidine-2,6-dione;

46. 3-(1-amino-4-oxo-4*H*-thiophene[3,4-*c*]pyrrole-5(6*H*)-yl)-1-(3 -methoxypropyl)piperidine-2,6-dione;

47. 3-(1-amino-4-oxo-4*H*-thiophene[3,4-*c*]pyrrole-5(6*H*)-yl)-1-isopropyl piperidine-2,6-dione;

48. 3-(1-amino-4-oxo-4*H*-thiophene[3,4-*c*]pyrrole-5(6*H*)-yl)-1-(2 -hydroxypropyl)piperidine-2,6-dione;

49. 3-(1-amino-4-oxo-4*H*-thiophene[3,4-*c*]pyrrole-5(6*H*)-yl)-1-(2 -methoxypropyl)piperidine-2,6-dione;

50. 3-(1-amino-4-oxo-4*H*-thiophene[3,4-*c*]pyrrole-5(6*H*)-yl)-1-butyl piperidine-2,6-dione;

51. 3-(1-methylamino-4-oxo-4*H*-thiophene[3,4-*c*]pyrrole-5(6*H*)-yl)-1 -methylpiperidine-2,6-dione;

52. 3-(1-methylamino-4-oxo-4*H*-thiophene[3,4-*c*]pyrrole-5(6*H*)-yl)-1 -ethyl piperidine-2,6-dione;

53. 3-(1-methylamino-4-oxo-4*H*-thiophene[3,4-*c*]pyrrole-5(6*H*)-yl)-1-(2 -methoxy-ethyl)piperidine-2,6-dione;

54. (*R*)-3-(1-amino-4-oxo-4*H*-thiophene[3, 4-*c*]pyrrole-5(6*H*)-yl)-3 -methyl-1-ethyl piperidine-2,6-dione;

55. (*S*)-3-(1-amino-4-oxo-4*H*-thiophene[3,4*-c*]pyrrole-5(6*H*)-yl)-3 -methyl-1-ethyl piperidine-2,6-dione;

56. (±)-3-(1-amino-4-oxo-4*H*-thiophene[3,4*-c*]pyrrole-5(6*H*)-yl)-3 -methyl-1-ethyl piperidine-2,6-dione;

57. (*R*)-3-(1-amino-4-oxo-4*H*-thiophene[3,4*-c*]pyrrole-5(6*H*)-yl)-3 -methyl-1-propyl piperidine-2,6-dione;

58. (*S*)-3-(1-amino-4-oxo-4*H*-thiophene[3,4-*c*]pyrrole-5(6*H*)-yl)-3 -methyl-1-propyl piperidine-2,6-dione;

59. (±)-3-(1-amino-4-oxo-4*H*-thiophene[3,4*-c*]pyrrole-5(6*H*)-yl)-3 -methyl-1-propyl piperidine-2,6-dione;

60. (*R*)-3-(1-amino-4-oxo-4*H*-thiophene[3,4*-c*]pyrrole-5(6*H*)-yl)-3 -methyl-1-(2-hydroxyethyl)piperidine-2,6-dione;

61. (*S*)-3-(1-amino-4-oxo-4*H*-thiophene[3,4*-c*]pyrrole-5(6*H*)-yl)-3 -methyl-1-(2-hydroxyethyl)piperidine-2,6-dione;

62. (±)-3-(1-amino-4-oxo-4*H*-thiophene[3,4*-c*]pyrrole-5(6*H*)-yl)-3 -methyl-1-(2-hydroxyethyl)piperidine-2,6-dione;

63. (*R*)-3-(1-amino-4-oxo-4*H*-thiophene[3, 4*-c*]pyrrole-5(6*H*)-yl)-3 -methyl-1-(2-methoxy-ethyl)piperidine-2,6-dione;

64. (*S*)-3-(1-amino-4-oxo-4*H*-thiophene[3,4*-c*]pyrrole-5(6*H*)-yl)-3 -methyl-1-(2-methoxy-ethyl)piperidine-2,6-dione;

65. (±)-3-(1-amino-4-oxo-4*H*-thiophene[3,4*-c*]pyrrole-5(6*H*)-yl)-3 -methyl-1-(2-methoxy-ethyl)piperidine-2,6-dione;

66. 3-(4-oxo-4*H*-thiophene[3,4-*c*]pyrrole-5(6*H*)-yl)-1 -(methoxycarbonylmethyl)piperidine-2,6-dione;

67. 3-(4-oxo-4*H*-thiophene[3,4-*c*]pyrrole-5(6*H*)-yl)-1 -(ethoxycarbonylmethyl)piperidine-2,6-dione;

68. 3-(1-amino-4-oxo-4*H*-thiophene[3,4-*c*]pyrrole-5(6*H*)-yl)-1 -(methoxycarbonylmethyl)piperidine-2,6-dione;

69. 3-(1-amino-4-oxo-4*H*-thiophene[3,4-*c*]pyrrole-5(6*H*)-yl)-1 -(ethoxycarbonylm ethyl )piperidine-2,6-dione;

70. 3-(1-amino-4-oxo-4*H*-thiophene[3,4-*c*]pyrrole-5(6*H*)-yl)-1-(methoxy carbonyl ethyl)piperidine-2,6-dione;

71. 3-(1-amino-4-oxo-4*H*-thiophene[3,4-*c*]pyrrole-5(6*H*)-yl)-1 -(ethoxycarbonyl ethyl)piperidine-2,6-dione;

72. (*R*)-3-(1-amino-4-oxo-4*H*-thiophene[3, 4*-c*]pyrrole-5(6*H*)-yl)-3 -methyl-1-hydroxymethyl-piperidine-2,6-dione;

73. (*S*)-3-(1-amino-4-oxo-4*H*-thiophene[3,4*-c*]pyrrole-5(6*H*)-yl)-3 -methyl-1-hydroxym ethyl-piperid ine-2,6-d ione;

74. (±)-3-(1-amino-4-oxo-4*H*-thiophene[3,4*-c*]pyrrole-5(6*H*)-yl)-3 -methyl-1-hydroxymethyl-piperidine-2,6-dione;

75. (*R*)-3-(1-amino-4-oxo-4*H*-thiophene[3,4-*c*]pyrrole-5(6*H*)-yl)-3-fluoro -1-hydroxymethyl-piperidine-2,6-dione;

76. (*S*)-3-(1-amino-4-oxo-4*H*-thiophene[3,4*-c*]pyrrole-5(6*H*)-yl)-3-fluoro -1-hydroxymethyl-piperidine-2,6-dione;

77. (±)-3-(1-amino-4-oxo-4*H*-thiophene[3,4*-c*]pyrrole-5(6*H*)-yl)-3-fluoro -1-hydroxymethyl-piperidine-2,6-dione;

78. (*R*)-3-(1-amino-4-oxo-4*H*-thiophene[3,4-*c*]pyrrole-5(6*H*)-yl)-3 -fluoropiperidine-2,6-dione;

79. (*S*)-3-(1-amino-4-oxo-4*H*-thiophene[3,4-*c*]pyrrole-5(6*H*)-yl)-3 -fluoropiperidine-2,6-dione

80. (±)-3-(1-amino-4-oxo-4*H*-thiophene[3,4*-c*]pyrrole-5(6*H*)-yl)-3 -fluoropiperidine-2,6-dione;

81. (*R*)-3-(4-oxo-4*H-*thiophene[3,4*-c*]pyrrole-5(6*H*)-yl)-3 -fluoropiperidine-2,6-dione;

82. (*S*)-3-(4-oxo-4*H*-thiophene[3,4-*c*]pyrrole-5(6*H*)-yl)-3 -fluoropiperidine-2,6-dione;

83. (±)-3-(4-oxo-4*H*-thiophene[3,4*-c*]pyrrole-5(6*H*)-yl)-3 -fluoropiperidine-2,6-dione;

84. (*R*)-3-(1-amino-4-oxo-4*H*-thiophene[3,4*-c*]pyrrole-5(6*H*)-yl)-3-fluoro -1-methylpiperidine-2,6-dione;

85. (*S*)-3-(1-amino-4-oxo-4*H*-thiophene[3,4-*c*]pyrrole-5(6*H*)-yl)-3-fluoro -1-methylpiperidine-2,6-dione;

86. (±)-3-(1-amino-4-oxo-4*H*-thiophene[3,4-*c*]pyrrole-5(6*H*)-yl)-3-fluoro -1-methylpiperidine-2,6-dione;

87. (*R*)-3-(1-amino-4-oxo-4*H*-thiophene[3,4-*c*]pyrrole-5(6*H*)-yl)-3-fluoro -1-ethyl piperidine-2,6-dione;

88. (*S*)-3-(1-amino-4-oxo-4*H*-thiophene[3,4*-c*]pyrrole-5(6*H*)-yl)-3-fluoro -1-ethyl piperidine-2,6-dione;

89. (±)-3-(1-amino-4-oxo-4*H*-thiophene[3,4-*c*]pyrrole-5(6*H*)-yl)-3-fluoro -1-ethyl piperidine-2,6-dione;

90. (*R*)-3-(1-amino-4-oxo-4*H*-thiophene[3,4-*c*]pyrrole-5(6*H*)-yl)-3-fluoro -1-propyl piperidine-2,6-dione;

91. (*S*)-3-(1-amino-4-oxo-4*H*-thiophene[3,4*-c*]pyrrole-5(6*H*)-yl)-3-fluoro -1-propyl piperidine-2,6-dione;

92. (±)-3-(1-amino-4-oxo-4*H*-thiophene[3,4*-c*]pyrrole-5(6*H*)-yl)-3-fluoro -1-propyl piperidine-2,6-dione;

93. (*R*)-3-(1-amino-4-oxo-4*H*-thiophene[3,4-*c*]pyrrole-5(6*H*)-yl)-3-fluoro -1-(2-methoxy-ethyl)piperidine-2,6-dione;

94. (*S*)-3-(1-amino-4-oxo-4*H*-thiophene[3,4-*c*]pyrrole-5(6*H*)-yl)-3-fluoro -1-(2-methoxy-ethyl)piperidine-2,6-dione;

95. (±)-3-(1-amino-4-oxo-4*H*-thiophene[3,4-*c*]pyrrole-5(6*H*)-yl)-3-fluoro -1-(2-methoxy-ethyl)piperidine-2,6-dione;

96. (*R*)-3-(4-oxo-4*H*-thiophene[3,4*-c*]pyrrole-5(6*H*)-yl)-3-fluoro-1 -hydroxymethyl-piperidine-2,6-dione;

97. (*S*)-3-(4-oxo-4*H*-thiophene[3, 4-*c*]pyrrole-5(6*H*)-yl)-3-fluoro-1 -hydroxymethyl-piperidine-2,6-dione;

98. (±)-3-(4-oxo-4*H*-thiophene[3, 4-*c*]pyrrole-5(6*H*)-yl)-3-fluoro-1 -hydroxymethyl-piperidine-2,6-dione;

99. (*R*)-3-(4-oxo-4*H*-thiophene[3, 4-*c*]pyrrole-5(6*H*)-yl)-3-fluoro-1 -methylpiperidine-2,6-dione;

100. (*S*)-3-(4-oxo-4*H*-thiophene[3, 4-*c*]pyrrole-5(6*H*)-yl)-3-fluoro-1 -methylpiperidine-2,6-dione;

101. (±)-3-(4-oxo-4*H*-thiophene[3, 4-*c*]pyrrole-5(6*H*)-yl)-3-fluoro-1 -methylpiperidine-2,6-dione;

102. (*R*)-3-(4-oxo-4*H*-thiophene[3, 4-*c*]pyrrole-5(6*H*)-yl)-3-fluoro-1-ethyl piperidine-2,6-dione;

103. (*S*)-3-(4-oxo-4*H*-thiophene[3, 4-*c*]pyrrole-5(6*H*)-yl)-3-fluoro-1-ethyl piperidine-2,6-dione;

104. (±)-3-(4-oxo-4*H*-thiophene[3, 4-*c*]pyrrole-5(6*H*)-yl)-3-fluoro-1-ethyl piperidine-2,6-dione;

105. (*R*)-3-(4-oxo-4*H*-thiophene[3, 4*-c*]pyrrole-5(6*H*)-yl)-3-fluoro-1 -propyl piperidine-2,6-dione;

106. (*S*)-3-(4-oxo-4*H*-thiophene[3, 4*-c*]pyrrole-5(6*H*)-yl)-3-fluoro-1 -propyl piperidine-2,6-dione;

107. (±)-3-(4-oxo-4*H*-thiophene[3, 4-*c*]pyrrole-5(6*H*)-yl)-3-fluoro-1 -propyl piperidine-2,6-dione;

108. (*R*)-3-(4-oxo-4*H*-thiophene[3, 4-*c*]pyrrole-5(6*H*)-yl)-3-fluoro-1-(2 -methoxy-ethyl)piperidine-2,6-dione;

109. (*S*)-3-(4-oxo-4*H*-thiophene[3, 4-*c*]pyrrole-5(6*H*)-yl)-3-fluoro-1-(2 -methoxy-ethyl)piperidine-2,6-dione;

110. (±)-3-(4-oxo-4*H*-thiophene[3, 4-*c*]pyrrole-5(6*H*)-yl)-3-fluoro-1-(2 -methoxy-ethyl)piperidine-2,6-dione;

111. (*R*)-3-(1-hydroxy-4-oxo-4*H*-thiophene[3,4*-c*]pyrrole-5(6H)-yl)-3 -fluoropiperidine-2,6-dione;

112. (*S*)-3-(1-hydroxy-4-oxo-4*H*-thiophene[3,4*-c*]pyrrole-5(6H)-yl)-3 -fluoropiperidine-2,6-dione;

113. (±)-3-(1-hydroxy-4-oxo-4*H*-thiophene[3,4*-c*]pyrrole-5(6H)-yl)-3 -fluoropiperidine-2,6-dione;

114. (*R*)-3-(1-methoxy-4-oxo-4*H*-thiophene[3,4*-c*]pyrrole-5(6*H*)-yl)-3 -fluoropiperidine-2,6-dione;

115. (*S*)-3-(1-methoxy-4-oxo-4*H*-thiophene[3,4*-c*]pyrrole-5(6*H*)-yl)-3 -fluoropiperidine-2,6-dione;

116. (±)-3-(1-methoxy-4-oxo-4*H*-thiophene[3,4*-c*]pyrrole-5(6*H*)-yl)-3 -fluoropiperidine-2,6-dione;

117. (*R*)-3-(1-acetylamino-4-oxo-4*H*-thiophene[3,4-*c*]pyrrole-5(6*H*)-yl) -3-fluoropiperidine-2,6-dione;

118. (*S*)-3-(1-acetylamino-4-oxo-4*H*-thiophene[3,4*-c*]pyrrole-5(6*H*)-yl) -3-fluoropiperidine-2,6-dione;

119. (±)-3-(1-acetylamino-4-oxo-4*H*-thiophene[3,4*-c*]pyrrole-5(6*H*)-yl)-3 -fluoropiperidine-2,6-dione;

120. (*R*)-3-(1-hydroxy-4-oxo-4*H*-thiophene[3,4-*c*]pyrrole-5(6*H*)-yl)-3 -methylpiperidine-2,6-dione;

121. (*S*)-3-(1-hydroxy-4-oxo-4*H*-thiophene[3,4*-c*]pyrrole-5(6*H*)-yl)-3 -methylpiperidine-2,6-dione;

122. (±)-3-(1-hydroxy-4-oxo-4*H*-thiophene[3,4*-c*]pyrrole-5(6*H*)-yl)-3 -methylpiperidine-2,6-dione;

123. (*R*)-3-(1-methoxy-4-oxo-4*H*-thiophene[3,4*-c*]pyrrole-5(6*H*)-yl)-3 -methylpiperidine-2,6-dione;

124. (*S*)-3-(1-methoxy-4-oxo-4*H-*thiophene[3,4-*c*]pyrrole-5(6*H*)-yl)-3 -methylpiperid ine-2,6-dione;

125. (±)-3-(1-methoxy-4-oxo-4*H*-thiophene[3,4*-c*]pyrrole-5(6*H*)-yl)-3 -methylpiperidine-2,6-dione;

126. 2-(2,6-dioxo-3-(4-oxo-4*H*-thiophene[3, 4*-c*]pyrrole-5(6*H*) -yl)piperidine-1-yl)ethyl nicotinate;

127. 2-(2,6-dioxo-3-(4-oxo-4*H*-thiophene[3, 4*-c*]pyrrole-5(6*H*) -yl)piperidine-1-yl) ethyl isonicotinate;

128. 2-(2,6-dioxo-3-(4-oxo-4*H*-thiophene[3, 4*-c*]pyrrole-5(6*H*) -yl)piperidine-1-yl) ethyl glycine ester;

129. 2-(2,6-dioxo-3-(4-oxo-4*H*-thiophene[3, 4*-c*]pyrrole-5(6*H*) -yl)piperidine-1-yl) ethyl alanine ester;

130. 2-(2,6-dioxo-3-(4-oxo-4*H*-thiophene[3, 4*-c*]pyrrole-5(6*H*) -yl)piperidine-1-yl) ethyl valine ester;

131. 2-(2,6-dioxo-3-(4-oxo-4*H*-thiophene[3, 4*-c*]pyrrole-5(6*H*) -yl)piperidine-1-yl) ethyl leucine ester;

132. 2-(2,6-dioxo-3-(4-oxo-4*H*-thiophene[3, 4*-c*]pyrrole-5(6*H*) -yl)piperidine-1-yl) ethyl isoleucine ester;

133. 2-(2,6-dioxo-3-(4-oxo-4*H*-thiophene[3, 4*-c*]pyrrole-5(6*H*) -yl)piperidine-1-yl) ethyl phenylalanine ester;

134. 2-(2,6-dioxo-3-(4-oxo-4*H*-thiophene[3, 4*-c*]pyrrole-5(6*H*) -yl)piperidine-1-yl) ethyl proline ester;

135. 2-(2,6-dioxo-3-(4-oxo-4*H*-thiophene[3, 4*-c*]pyrrole-5(6*H*) -yl)piperidine-1-yl)ethyl dimethylamino acetate;

136. 2-(2,6-dioxo-3-(4-oxo-4*H*-thiophene[3, 4*-c*]pyrrole-5(6*H*) -yl)piperidine-1-yl)ethyl methylamino acetate;

137. 2-(2,6-dioxo-3-(4-oxo-4*H*-thiophene[3, 4*-c*]pyrrole-5(6*H*) -yl)piperidine-1-yl)ethyl diethylamino acetate;

138. 2-(2,6-dioxo-3-(4-oxo-4*H*-thiophene[3, 4*-c*]pyrrole-5(6*H*) -yl)piperidine-1-yl)ethyl ethylamino acetate;

139. 2-(2,6-dioxo-3-(4-oxo-4*H*-thiophene[3, 4*-c*]pyrrole-5(6*H*) -yl)piperidine-1-yl)ethyl 1-piperidyl acetate;

140. 2-(2,6-dioxo-3-(4-oxo-4*H*-thiophene[3, 4*-c*]pyrrole-5(6*H*) -yl)piperidine-1-yl)ethyl 1-pyrrolinyl acetate;

141. (*R*)-3-(1-amino-4-oxo-4*H*-thiophene[3,4*-c*]pyrrole-5(6*H*) -yl)piperidine-2-one;

142. (*S*)-3-(1-amino-4-oxo-4*H*-thiophene[3,4*-c*]pyrrole-5(6*H*) -yl)piperidine-2-one;

143. (±)-3-(1-amino-4-oxo-4*H*-thiophene[3,4*-c*]pyrrole-5(6*H*) -yl)piperidine-2-one;

144. (*R*)-5-(1-amino-4-oxo-4*H*-thiophene[3,4*-c*]pyrrole-5(6*H*) -yl)piperidine-2-one;

145. (*S*)-5-(1-amino-4-oxo-4*H-*thiophene[3,4*-c*]pyrrole-5(6*H*) -yl)piperidine-2-one;

146. (±)-5-(1-amino-4-oxo-4*H*-thiophene[3,4*-c*]pyrrole-5(6*H*) -yl)piperidine-2-one;

147. (*R*)-3-(4-oxo-4*H*-thiophene[3,4-*c*]pyrrole-5(6*H*)-yl)piperidine-2 -one;

148. (*S*)-3-(4-oxo-4*H*-thiophene[3,4-*c*]pyrrole-5(6*H*)-yl)piperidine-2 -one;

149. (±)-3-(4-oxo-4*H*-thiophene[3,4*-c*]pyrrole-5(6*H*)-yl)piperidine-2 -one;

150. (*R*)-5-(4-oxo-4*H*-thiophene[3,4*-c*]pyrrole-5(6*H*)-yl)piperidine-2 -one;

151. (*S*)-5-(4-oxo-4*H*-thiophene[3,4-*c*]pyrrole-5(6*H*)-yl)piperidine-2-one; and

152. (±)-5-(4-oxo-4*H*-thiophene[3,4*-c*]pyrrole-5(6*H*)-yl)piperidine-2 -one.

The compound of Formula **(I)** or Formula (**II**) of the invention suitable for being used as medical active ingredients may be prepared in form of free bases or inorganic acid salts, comprising hydrochloride, sulfate, nitrate, phosphate, or in form of organic salts, comprising sulfonate, acetate, formate, fumarate, maleate, citrate, tartrate, malate, benzoate, ascorbate, gluconate, lactate, succinate, or trifluoroacetate.

In another embodiment of the invention, provided a method of preparing a compound of Formula **(I)** or Formula (**II**),

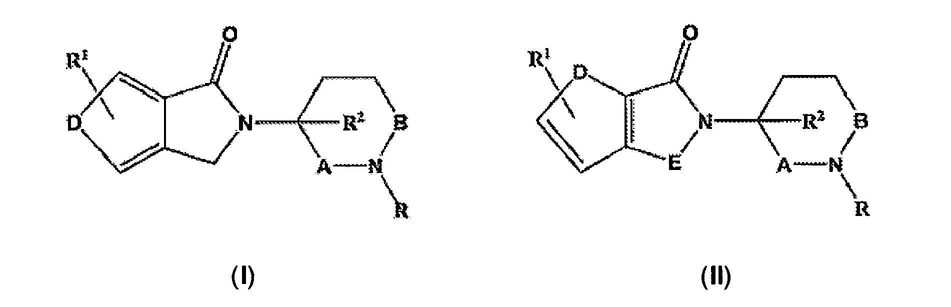

wherein

A, B and E independently represent CH₂, or CO;

D represents S, NH, or NC₁₋₆ alkylhydrocarbyl;

R represents H, C₁₋₆alkylhydrocarbyl, or R³;

R¹ at each occurrence independently represents H, or independently one or two occurrences of F, Cl, Br, C₁₋₄alkylhydrocarbyl, OH, OC₁₋₄alkylhydrocarbyl, NO₂, NHC(O)C₁₋₄alkylhydrocarbyl, NH₂, NH(C₁₋₄alkylhydrocarbyl), or N(C₁₋₄alkylhydrocarbyl)₂,

R² at each occurrence represents F, CF₃, H, or C_{1 -4}alkylhydrocarbyl;

R³ at each occurrence represents (CH₂)ₘR⁴, and m represents an integer from 1 to 6;

R⁴ represents F, Cl, H, C₁₋₄alkylhydrocarbyl, OH, OC, ₋₄alkylhydrocarbyl, NHC(O)C₁₋₄alkylhydrocarbyl, NH₂, NH(C_{1 -4}alkylhydrocarbyl), N(C₁₋₄alkylhydrocarbyl)₂, or O₂CR⁵;

R⁵ represents CHR⁶NR⁷R⁸, CHR⁶NR⁹C(O)CHR¹⁰NR⁷R⁸, heterocycle W, or CHR⁶NR⁹C(O)W;

R⁶, R⁹ and R¹⁰ independently represent H, or C_{1 -4}alkylhydrocarbyl;

R⁷ and R⁸ independently represents H, C₁₋₄alkylhydrocarbyl or taken together in combination represent 1,3-propylidene, 1,4-butylene, 1,5-pentylene, or 1,6-hexylidene, and

W represents saturated or unsaturated four to eight-membered heterocycle.

the method comprising the steps of:

(1) contacting a compound of Formula (**VII**), Formula (**VIII**), or Formula (**IX**) with a compound of Formula **(X)** to obtain a compound of Formula (**XI**) or Formula (**XII**),

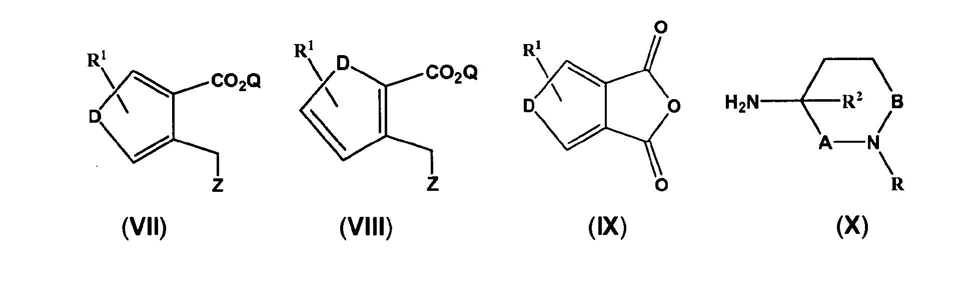

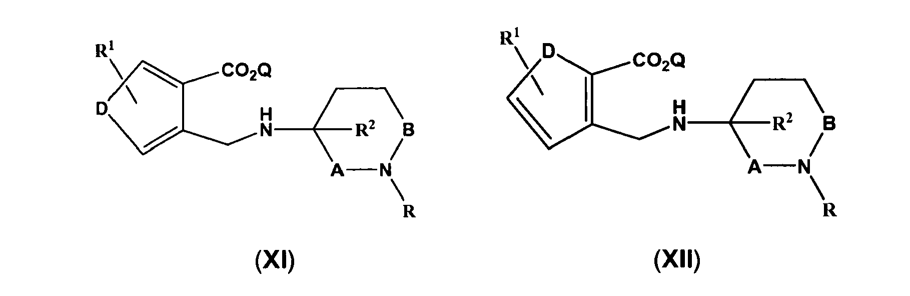

wherein the definitions of A, B, D, R, R¹, R² are the same as those for Formula **(I)** or Formula (**II**), Z represents Cl, Br, I, Ms, Ts, and Q represents methyl, or tert-butyl.

In a class of this embodiment, the molar ratio of the compound of Formula (**VII**), Formula (**VIII**), or Formula (**IX**) to the compound of Formula (**X**) is between 3:1 and 1:3. The reaction is facilitated by an inorganic base including but not limited to NaH, KH, CaH₂, K₂CO₃, Na₂CO₃, KHCO₃, NaHCO₃, Li₂CO₃, Cs₂CO₃, LiOH, KOH, NaOH, Ca(OH)₂, K₃PO₄, K₂HPO₄, or an organic base. The proportion of base is between 50 % and 300% by mole.

The reaction is conducted in an organic solvent, such as dichloromethane, chloroform, acetone, butanone, dimethylformamide, dimethylsulfoxide, ethylene glycol dimethyl ether, tetrahydrofuran, pyridine, or acetonitrile, and may be conducted under multi-phase conditions, especially at presence of a phase-transfer catalyst.

(2) hydrolyzing the compound of Formula (**XI**) or Formula (**XII**) to obtain a corresponding acid of Formula (**XIII**) or Formula (**XIV**), and

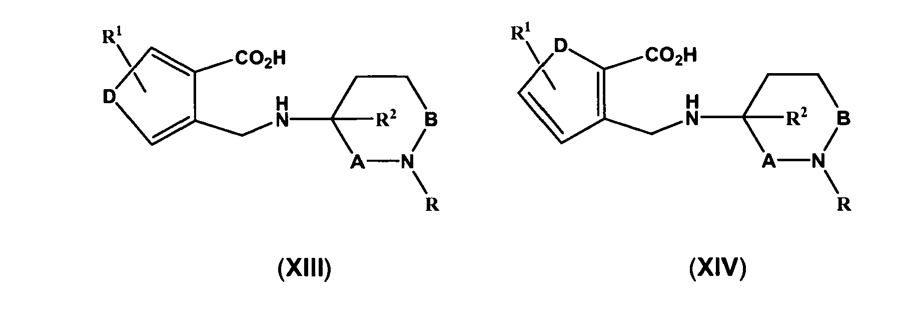

(3) dehydrating and cyclizing the compound of Formula (**XIII**) or Formula (**XIV**) to obtain the compound of Formula **(I)** or Formula (**II**).

The reactions are conducted in an organic solvent, such as dichloromethane, chloroform, acetone, butanone, dimethylformamide, dimethylsulfoxide, ethylene glycol dimethyl ether, tetrahydrofuran, pyridine or acetonitrile. A condensing agent such as thionyl chloride, DCC, CDI, EDCI may be added, and pyridine derivatives such as DMAP, 4-(1-pyrroline) pyridine may be added as catalyst.

Another method of preparing a compound of Formula **(I)** or Formula (**II**) comprising contacting a compound of Formula (**XV**) or Formula (**XVI**) with a compound of formula L-R to obtain a compound of Formula (**I**) or Formula (**II**), wherein the definition of A, B, D, E, R, R¹, R² are the same as that for Formula (**I**) or Formula (**II**), L represents Cl, Br, I, Ms or Ts, and R is the same as that for Formula (**I**) or Formula (**II**).

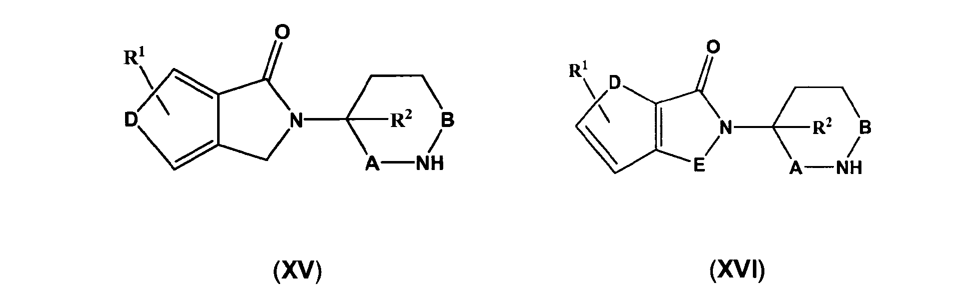

The molar ratio of the compound of Formula (**XV**) or Formula (**XVI**) to the compound of formula L-R is between 3:1 and 1:3. The reaction may be facilitated by an inorganic base, including but not limited to NaH, KH, CaH₂, K₂CO₃, Na₂CO₃, KHCO₃, NaHCO₃, Li₂CO₃, Cs₂CO₃, LiOH, KOH, NaOH, Ca(OH)_{2,} K₃PO₄, K₂HPO₄, or an organic base. The proportion of the base to the compound of formula L-R is between 50 % and 300 % by mole.

The reactions are conducted in an organic solvent, such as dichloromethane, chloroform, acetone, butanone, dimethylformamide, dimethylsulfoxide, ethylene glycol dimethyl ether, tetrahydrofuran, pyridine or acetonitrile, and may be conducted under multi-phase conditions, especially at presence of a phase-transfer catalyst.

In another embodiment of the invention, provided is use of a compound of Formula **(I)** or Formula (**II**) as medical active ingredient,

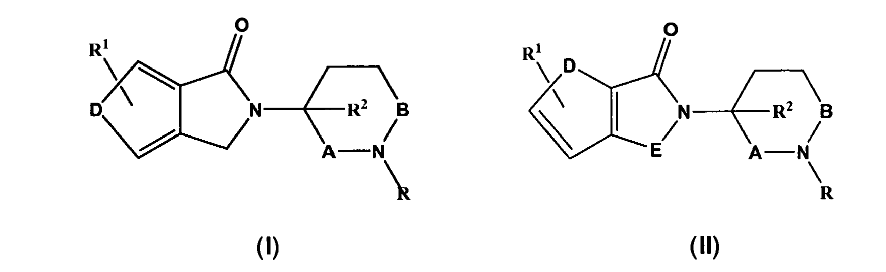

wherein

A, B and E independently represent CH₂, CO;

D represents S, NH, or NC₁₋₆ alkylhydrocarbyl;

R represents H, C₁₋₆alkylhydrocarbyl, or R³;

R¹ at each occurrence independently represents H, or independently one or two same or different occurrences of F, Cl, Br, C_{1 -4}alkylhydrocarbyl, OH, OC₁₋₄alkylhydrocarbyl, NO₂, NHC(OC_{1 -4}alkylhydrocarbyl, NH₂, NH(C₁₋₄alkylhydrocarbyl), or N(C_{1 -4}alkylhydrocarbyl)₂;

R² at each occurrence represents F, CF₃, H, or C₁ -₄alylhydrocarbyl;

R³ at each occurrence represents (CH₂)ₘR⁴, and m represents an integer from 1 to 6;

R⁴ represents F, Cl, H, C₁₋₄alkylhydrocarbyl, OH, OC_{1 -4}alkylhydrocarbyl, NHC(O)C₁₋₄alkylhydrocarbyl, NH₂, NH(C_{1 -4}alkylhydrocarbyl), N(C₁₋₄alkylhydrocarbyl)₂, or O₂CR⁵;

R⁵ represents CHR⁶NR⁷R⁸, CHR⁶NR⁹C(O)CHR¹⁰NR⁷R⁸, heterocycle W, or CHR⁶NR⁹C(O)W_{;}

R⁶, R⁹, and R¹⁰ independently represents H, or C_{1 -4}alkylhydrocarbyl;

R⁷ and R⁸ independently represent H, C₁₋₄alkylhydrocarbyl or taken together in combination represent 1,3-propylidene, 1,4-butylene, 1,5-pentylene, or 1,6-hexylidene, and

W represents a saturated or unsaturated four to eight-membered heterocycle.

The diseases which can be effectively alleviated or treated by decreasing TNFα concentration in patients after administering the pharmaceutical composition comprising the compound of Formula **(I)** or Formula (**II**) include but are not limited to inflammatory diseases, infectious diseases, autoimmune diseases, or malignant tumors.

Specifically, the disease includes but is not limited to septic shock, endotoxic shock, hemodynamic shock, septic syndrome, post ischemic reperfusion injury, malaria, mycobacterial infection, meningitis, psoriasis, congestive heart failure, fibrotic disease, cachexia, transplant immune rejection, cancer, autoimmune disease, opportunistic infection in AIDS, erythema nodosum leprosy, lupus erythematosus, refractory lupus erythematosus, Behcet syndrome, regional ileitis, myelodysplastic syndrome, rheumatoid arthritis (RA), hepatitis, nephritis, rheumatoid spondylitis, multiple myeloma, thyroid tumor, kidney cancer, prostate cancer, lymphoma, leukemia, liver cancer, brain glioma, colorectal cancer, lung cancer, stomach cancer, breast cancer, melanoma, cervical cancer, pancreatic cancer, esophageal cancer, oral cancer, throat cancer, or rhinocarcinoma.

In another aspect, the invention provides a pharmaceutical composition comprising a compound of Formula **(I)** or Formula (**II**),

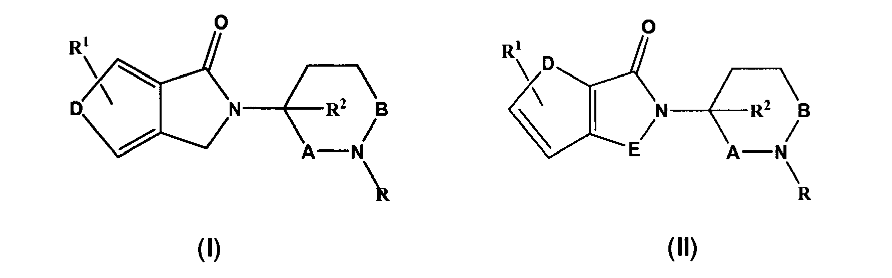

wherein

A, B and E independently represent CH₂, or CO;

D represents S, NH, or NC₁₋₆ alkylhydrocarbyl;

R represents H, C₁₋₆alkylhydrocarbyl, or R³;

R¹at each occurrence independently represents H, or independently one or two same or different occurrences of F, Cl, Br, C_{1 -4}alkylhydrocarbyl, OH, OC₁₋₄alkylhydrocarbyl, NO₂, NHC(O)C_{1 -4}alkylhydrocarbyl, NH₂, NH(C₁₋₄alkylhydrocarbyl), or N(C_{1 4}alkylhydrocarbyl)₂;

R² at each occurrence represents F, CF₃, H, or C_{1 -4}alkylhydrocarbyl;

R³ at each occurrence represents (CH₂)ₘR⁴, and m represents an integer from 1 to 6;

R⁴ represents F, Cl, H, C₁₋₄alkylhydrocarbyl, OH, OC_{1 -4}alkylhydrocarbyl, NHC(O)C₁₋₄alkylhydrocarbyl, NH₂, NH(C_{1 -4}alkylhydrocarbyl), N(C₁₋₄alkylhydrocarbyl)₂, or O₂CR⁵;

R⁵ represents CHR⁶NR⁷R⁸, CHR⁶NR⁹C(O)CHR¹⁰NR⁷R⁸, heterocycle W, or CHR⁶NR₉C(O)W_{;}

R⁶, R⁹ and R¹⁰ independently represents H, or C_{1 -4}alkylhydrocarbyl;

R⁷ and R⁸ independently represent H, C₁₋₄alkylhydrocarbyl or taken together in combination represent 1,3-propylidene, 1,4-butylene, 1,5-pentylene, or 1,6-hexylidene, and

W represents saturated or unsaturated four to eight-membered heterocycle.

In a class of this embodiment, the pharmaceutical composition further comprises a pharmaceutically acceptable carrier, excipient, filler, solvent, diluent, coloring agent, or adhesive. The type and dosage of these additives depends on an administration mode of the pharmaceutical composition.

The mode of administration of the pharmaceutical composition is selected from gastrointestinal administration, intravenous injection, intraperitoneal injection, dermal injection, intramuscular injection, intranasal administration, intraocular administration, inhalation, rectal administration, reproductive tract administration, percutaneous absorption, or other drug delivery methods.

The pharmaceutical composition comprising the compound of Formula **(I)** or Formula **(II)** may be used in combination with another pharmaceutically acceptable composition.

Pharmacological Research: Effects on the releasing of TNFa in peripheral blood mononuclear cells (PBMCs) stimulated by lipopolysaccharide (LPS).

Cytokine TNFα released by PBMCs in the peripheral blood stimulated by lipid polysaccharide (LPS) in vitro was studied. Experiments of inhibition of the release of TNFα in peripheral blood mononuclear cells (PBMCs) stimulated by lipopolysaccharide (LPS) by compounds of Formula (I) or Formula (II) are described below:

PBMCs were collected from blood of at least three volunteers pretreated with heparin by a gradient separation method, and washed three times with a 1640 culture medium (10% calf serum, 2 mM L-glutamine, 100 mM mercaptoethanol, 50µg/mL streptomycin, and 50 U/mL penicillin).The obtained PBMCs were then placed into a 24-well cell culture plate and the concentration was adjusted to 1x10⁶ cells/mL with 1640 culture medium. The compounds to be tested were dissolved in dimethylsulfoxide to obtain a solution having a required concentration. The solution was added to the above -mentioned cell culture medium and cultured in an incubator (5% CO₂, 90% humidity) for 1 hour. Then, LPS (Sigma) was added until the concentration reached 0.1 µg/mL (except for the control).

After 20 hours incubation, the content of TNFα in the supernatant of the PBMC culture medium was assayed by ELISA kit (America Genzyme Co) using standard method. The TNFα inhibition rate was calculated by measured value of the control well (not treated) and the measured value of the test wells (treated with the test compound). The concentration of compounds giving a 50% TNFα inhibition (IC50 value) was calculated using nonlinear regression analysis. Each concentration was determined twice and an average value was practicable. Results are listed in Table **1**.

**Table 1. Inhibition of the release of TNFα in peripheral blood mononuclear cells (PBMCs) stimulated by (LPS) by compounds listed**

| **Compound** | **Concentration (µm)** | **Inhibition Degree (%)** | **EC₅₀ (µM)** |
|---|---|---|---|
| Thalidomide | 100 | 22 | 183 |
| Example 9 | 3.0 | 32 | |
| Example 10 | 3.0 | 28 | |
| Example 11 | 3.0 | 22 | |
| Example 12 | 3.0 | NA | |
| Example 16 | 3.0 | 38 | |
| Example 17 | 3.0 | 19 | |
| Example 18 | 3.0 | 94 | 0.06 |
| Example 20 | 3.0 | 76 | 0.18 |
| Example 33 | 3.0 | 47 | |
| Example 34 | 3.0 | 18 | |

Abbreviations

CDI: carbonyl diimidazole; DCM: dichloromethane; DCE: 1,2-dichloroethane, THF: tetrahydrofuran, TFA: trifluoroacetic acid; DMAP: 4 -(*N,N*-dimethylamino)pyridine; TEA: triethylamine; DMF: *N,N* -dimethylformamide; DMSO: dimethyl sulfoxide.

### Example 1

### 3,4-Dicyanothiophene

To a 2000 mL three-necked flask, equipped with a mechanic stirrer, a reflux condenser, and an inert gas duct, 96.8 g of 3,4-dibromothiophene, 104 g of cuprous cyanide, and 100 mL of dry DMF were added. After refluxing for 4h, the reaction mixture was cooled down to room temperature; and a solution obtained by dissolving 400 g of FeCl₃•6H₂O in 700 mL of hydrochloric acid (1.7 M) was added into the reaction mixture and allowed to react for 30 min at between 60 and 70°C. After the reaction mixture was fully cooled, 500 mL DCM was added. The reaction mixture was divided into 300 mL portions and each portion extracted with DCM (2×300 mL). The DCM layers were combined. The extracts were divided into 600 mL portions, washed successively with 2×50 mL 6N hydrochloric acid, water, saturated Na₂CO₃ solution, and brine; dried over anhydrous MgSO₄, filtered, and evaporated to obtain a yellow solid. The solid was washed with a mixture of ethyl acetate : petroleum ether = 1:1, and filtered to obtain a white solid (21 g). ¹H NMR (CDCl₃): δ 8.07 (s, 2H).

### Example 2

### Thiophene-3,4-dicarboxylic acid

To a 500 mL round bottom flask equipped with an electromagnetic stirrer, and a reflux condenser, 15.978 g of 3,4-dicyanothiophene, 43.997 g of KOH, and 174 mL of glycol were added; and the mixture was refluxed for 4h. After the reaction mixture was cooled, 350 mL of water was added, and the aqueous layer was extracted with diethyl ether (2×100 mL). The ether layer was removed, the aqueous layer was cooled down in an ice bath, and an excess of strong hydrochloric acid was added until a white precipitate appeared. The solid was filtered and dissolved in 2000 mL of ether. The filtrate was extracted with diethyl ether (3×300 mL). Organic layers were combined, dried over anhydrous MgSO₄, filtered, and evaporated. 15 g of white solid was obtained and recrystallized from water. ¹H NMR (DMSO-d₆): δ 10.35 (brs, 2H), 8.17 (s, 2H); MS (m/z): 171 (M-1)⁺.

### Example 3

### Thiophene-[3, 4-c]furan-1,3-dione

To a 250 mL round bottom flask, equipped with an electromagnetic stirrer, a reflux condenser, and a dying tube, 15 g of thieno-3,4-dicarboxylic acid and 120 mL of acetic anhydride were added. The mixture was refluxed for 3 h, evaporated to remove solvent to give 13 g of deep brown solid.

### Example 4

### 4-(tert-butoxycarbonyl) thiophene 3-carboxylic acid

To a 250 mL round bottom flask, equipped with an electromagnetic stirrer, and a drying tube, 15.4 g of thiophene [3, 4*-c*]furan-1,3-dione, 1.22 g of DMAP, 40 mL of tert-butyl alcohol, 18 mL of dry TEA, and 40 mL of DCM were added, and the reaction mixture was stirred overnight at room temperature. The solvent was evaporated, and 200 mL of CHCl₃ and 50 mL of water were added. The organic layer was washed successively with 1 N hydrochloric acid, water and saturated brine, dried over anhydrous MgSO₄, filtered, and evaporated to give a solid.

### Example 5

### Tert-butyl 4-(hydroxymethyl)-thiophene-3-carboxylate

To a 250 mL round bottom flask, equipped with an electromagnetic stirrer and a drying tube, 13.856 g of 4-(tert-butoxycarbonyl) thiophene 3 -carboxylic acid, 150 mL of dry THF, and 15.552 g of CDI were added, and the reaction mixture was stirred overnight at room temperature. The resultant solution was added dropwise to another solution prepared by dissolving 15.96 g of NaBH₄ in 90 mL of THF and 130 mL of water, and it was stirred for 30 minutes. 1 N hydrochloric acid was added to adjust the pH value to 5. THF was evaporated, and the solution was extracted with 200 mL of CHCl₃. The organic layer was washed successively with saturated NaHCO₃ solution, 1 N hydrochloric acid, water and saturated brine, dried over anhydrous MgSO₄, filtered, evaporated, and purified by column chromatography to give 13.74 g of solid. ¹H NMR (CDCl₃): δ 8.03 (d, 1H, J=3Hz), 7.17 (d, 1H, J=3Hz), 4.70 (s, 2H), 1.58 (s, 9H).

### Example 6

### Tert-butyl 4-(iodo methyl) thiophene-3-carboxylate

To a 500 mL three-necked flask, equipped with an electromagnetic stirrer, a constant pressure funnel, and an inert gas duct, 10.492 g of Ph₃P and 240 mL of dried DCM were added. Ten minutes of stirring, 10.172 g of iodine was added, and the mixture was stirred for additional 15 minutes. Afterwards, 3.094 g of imidazole was added. 3.428 g of tert-butyl 4-hydroxymethyl -thiophene-3-carboxylate was dissolved in 80 mL of dry DCM, and then transferred to a constant pressure funnel and added dropwise to the above -mentioned mixture. The resultant mixture was refluxed for an hour. The solution was cooled down, the organic layer was washed successively with 5% sodium thiosulfate twice, water once, and saturated brine once, dried over anhydrous MgSO₄, filtered, evaporated, and purified by column chromatography to give 3.556 g of oil-like products.

### Example 7

### Benzyl 2,6-dioxopiperidin-3-yl carbamate

11.2 g of CBZ-L-glutamine was dissolved in 120 mL of anhydrous THF. 7.776 g of CDI and a catalytic quantity of DMAP were added to the above solution. The reaction mixture was refluxed for 6h. After cooling, the reaction mixture was filtered to remove a small quantity of insoluble substances, evaporated to remove THF, and recrystallized from ethyl acetate to obtain a white solid (8.5 g). ¹H NMR (CDCl₃):δ 8.37 (s, 1H), 7.36-7.26 (m, 5H, Ph), 5.67 (d, 1H, J = 3Hz), 5.14 (s, 2H), 4.40-4.33 (m, 1H), 2.82-2.67 (m, 2H), 2.58-2.49 (m, 1H), 1.96-1.85 (m, 1H).

### Example 8

### 3-Aminopiperidine-2,6-dione

7.86 g of benzyl 2,6-dioxopiperidin-3-yl carbamate were dissolved in 30 mL of THF and 30 mL of methanol. 0.786 g of 10% Pd/C catalyst was added to the above solution. The mixture was allowed to react under a flow of hydrogen at room temperature for 2h, filtered to remove the catalyst, and evaporated to remove the solvent. A light blue solid (3.818 g) was obtained.

### Example 9

### 3-(4-oxo-4H-thiophene[3,4-c] pyrrol-5(6H)-yl) piperidine-2,6-dione

To a 50 mL round bottom flask, equipped with an electromagnetic stirrer and a drying tube, 1.736 g of 3-aminopiperidine-2,6-dione, 2.197 g of tert-butyl 4-(iodo methyl) thiophene-3-carboxylate, 1.871 g of K₂CO₃ and 20 mL of dry DMF were added. The mixture was stirred overnight at room temperature. Then, 200 mL of water was added, and the solution was extracted with ethyl acetate, washed successively with water and saturated brine, dried over anhydrous MgSO₄, filtered, evaporated, purified with column chromatography to give 1. 636 g of foam-like solid. The solid was dissolved in 15 mL of DCM and 5 mL of TFA, stirred overnight at room temperature. The solvent was evaporated, and the residues was dissolved in 20 mL of DCE, 4 mL of SOCl₂ was added, refluxed for 2 hours, cooled to give a pink solid. The solid was washed with water and THF successively to give 0.885 g of white solid. ¹H NMR (DMSO-d₆): δ 8.02 (d, 1H, J=2Hz), 7.50 (s, 1H), 5.01 (dd, 1H, J=2Hz, J=11Hz), 4.34 (d, 1H, J=12Hz), 4.19 (d, 1H, J=12Hz), 2.93-2.85 (m, 1H), 2.62 -2.47 (m, 1H), 2.41-2.31 (m, 1H), 2.03-1.98 (m, 1H); MS (*m*/*z*): 249 [M-1]⁺.

### Example 10

### 1-methyl-3-(4-oxo-4H-thiophene [3,4-c] pyrrol-5 (6H)-yl) piperidine-2,6-dione

0.250 g of 4-(4-oxo-4H-thiophene [3, 4-c] pyrrole-5 (6H)-yl) piperidine-2,6-dione was dissolved in 10 mL of dry DMF, and 0.036 g of NaH (95%) was added. After 30 minutes of reaction at room temperature, 0.2 mL of CH₃l was added, and the reaction mixture was allowed to stand overnight. Thereafter, 100 mL of water was added, and the mixture was extracted with ethyl acetate (30mLx3). Organic layers were combined, washed successively with 30 mL of water, saturated brine, dried over anhydrous MgSO₄, filtered, evaporated to give 0.176 g of white solid. ¹H NMR (DMSO-d₆): δ 7.98 (d, 1H, J=2Hz), 7.45 (d, 1H, J=1Hz), 5.03 (dd, 1H, J=4Hz, J=11Hz), 4.29 (d, 1H, J=12Hz), 4.14 (d, 1H, J=12Hz), 2.97 (s, 3H), 2.97-2.87 (m, 1H), 2.74-2.69 (m, 1H), 2.33-2.26 (m, 1H), 2.00-1.94 (m, 1H); MS (*m*/*z*): 263 [M-1]⁺.

### Example 11

### 3-(4-oxo-4H-thiophene [3,4-c] pyrrol-5 (6H)-yl)-1-propylpiperidine-2,6-dione

Following Example 10 and substituting iodomethane with bromopropane, 0.185 g of white solid was obtained. ¹H NMR (CDCl₃): δ 7.81 (d, 1H, J=2Hz), 7.13 (s, 1H), 5.10 (dd, 1H, J=3Hz, J=10Hz), 4.34 (d, 1H, J=12Hz), 4.25 (d, 1H, J=12Hz), 3.79-3.72 (m, 2H), 3.00-2.95 (m, 1H), 2.88 -2.79 (m, 1H), 2.33-2.16 (m, 2H), 1.59-1.51 (m, 2H), 0.90 (t, 3H, J=5Hz); MS (*m*/*z*): 293 [M+1]⁺.

### Example 12

### 1-cyclopentyl-3-(4-oxo-4H-thiophene [3,4-c] pyrrol-5 (6H)-yl) piperidine-2,6-dione

Following Example 10 and substituting iodomethane with bromopentane, 0.225 g of white solid was obtained. ¹H NMR (CDCl₃): δ 7.79 (d, 1H, J=2Hz), 7.11 (s, 1H), 5.10 (dd, 1H, J=4Hz, J=10Hz), 4.33 (d, 1H, J=12Hz), 4.24 (d, 1H, J=12Hz), 2.98-2.92 (m, 1H), 2.85-2.76 (m, 1H), 2.33 -2.03 (m, 2H), 1.90-1.51 (m, 8H).

### Example 13

### 1-methoxy carbonyl methyl-3-(4-oxo-4H-thiophene [3,4-c] pyrrole-5 (6H)-yl) piperidine-2,6-dione

Following Example 10 and substituting iodomethane with methyl bromoacetate, 0.195 g of white solid was obtained. ¹H NMR (CDCl₃): δ 7.81 (d, 1H, J=2Hz), 7.13 (s, 1H), 5.24 (dd, 1H, J=4Hz, J=12Hz), 4.62 (d, 1H, J=12Hz), 4.48 (d, 1H, J=12Hz), 4.33 (d, 1H, J=12Hz), 4.27 (d, 1H, J=12Hz), 3.75 (s, 3H), 3.06-2.85 (m, 2H), 2.42-2.18 (m, 2H); MS (*m*/*z*): 345 [M+Na]⁺.

### Example 14

### 1-(2-methoxy ethyl)-3-(4-oxo-4H-thiophene [3,4-c] pyrrole-5 (6H)-yl) piperidine-2,6-dione

Following Example 10 and substituting iodomethane with 2-bromo-ethyl methyl ether, and 0.237 g of white solid was obtained. ¹H NMR (CDCl₃): δ 7.81 (d, 1H, J=2Hz), 7.14 (s, 1H), 5.15 (dd, 1H, J=4Hz, J=10Hz), 4.34 (d, 1H, J=12Hz), 4.25 (d, 1H, J=12Hz), 4.04 (t, 2H, J=4Hz), 3.52 (t, 2H, J=4Hz), 3.33 (s, 3H), 3.02-2.81 (m, 2H), 2.30-2.13 (m, 2H); MS (*m*/*z*): 309 [M+1]⁺.

### Example 15

### 1-(2-(thiophene-2-yl) ethyl)-3-(4-oxo-4H-thiophene [3,4-c] pyrrole-5 (6H)-yl) piperidine-2,6-dione

Following Example 10 and substituting iodomethane with 2-(2-iodo ethyl) thiophene (which was prepared from 2-(thiophene-2-yl) ethanol according to the method of Example 6), 0.267 g of white solid was obtained. ¹H NMR (CDCl₃): δ 7.81 (d, 1H, J=2Hz), 7.15 (d, 1H, J=4Hz), 7.11 (s, 1H), 6.93 (dd, 1H, J=3Hz, J=4Hz), 6.83 (d, 1H, J=3Hz), 5.07 (dd, 1H, J=4Hz, J=10Hz), 4.21 (d, 1H, J=12Hz), 4.12 (d, 1H, J=12Hz), 4.09-4.04 (m, 2H), 3.09 (t, 2H, J=5Hz), 2.97-2.76 (m, 2H), 2.21-2.10 (m, 2H).

### Example 16

### 3-methyl-3-(4-oxo-4H-thiophene [3,4-c] pyrrole-5 (6H)-yl) piperidine-2,6-dione

To a 50 mL round bottom flask, equipped with an electromagnetic stirrer and a drying tube, 0.284 g of 3-amino-3-methyl-piperidine-2,6-dione (prepared by a method disclosed in Bioorg. Med. Chem. Lett. 1999, 9, 1625), 0.324 g of tert-butyl 4-(iodo methyl) thiophene-3-carboxylate, 0.276 g of K₂CO₃ and 10 mL of dry DMF were added. The mixture was stirred overnight at room temperature. Then 100 mL of water was added, the solution was extracted with ethyl acetate, washed successively with water and saturated brine, dried over anhydrous MgSO₄, filtered, evaporated, and purified by column chromatography to give a foam-like solid. The solid was dissolved in 6 mL of DCM and 2 mL of TFA, and stirred overnight at room temperature. The solvent was evaporated, and the residues were dissolved in 5 mL of DCE. 2 mL of SOCl₂ was added, and the mixture was refluxed for 2 hours, cooled and filtered to give a pink solid. The solid was washed with water and THF successively to give a white solid. MS (*m*/*z*): 263 [M-1]⁺.

### Example 17

### 3-(1-nitro-4-oxo-4H-thiophene [3,4-c] pyrrole-5 (6H)-yl) piperidine-2,6-dione

To a 50 mL round bottom flask, equipped with an electromagnetic stirrer and a drying tube, 5 mL of fuming nitric acid (95%) was added. The solution was cooled on an ice bath to a temperature between 0 and 5°C, and 4-(4-oxo -4H-thiophene [3,4-c] pyrrole-5 (6H)-yl) piperidine-2,6-dione was added. The reaction mixture was allowed to react for 10 minutes. The mixture was then poured into 50 g of ice and water mixture. The pH value of the resultant solution was adjusted to about 2 with 20% NaOH solution yielding a pink solid. The solid was washed successively with water and THF to give 0.152 g of white solid. ¹H NMR (DMSO-d₆): δ 8.43 (s, 1H), 4.98 (dd, 1H, J=4Hz, J=10Hz), 4.67 (d, 1H, J=14Hz), 4.52 (d, 1H, J=14Hz), 2.88-2.79 (m, 1H), 2.58-2.35 (m, 2H), 1.98-1.95 (m, 1H); MS (*m*/*z*): 294 (M-1]⁺.

### Example 18

### 3-(1-amino-4-oxo-4H-thiophene [3,4-c] pyrrole-5 (6H)-yl) piperidine-2,6-dione

0.102 g of 4-(1-nitro-4-oxo-4H-thiophene [3,4-c] pyrrole-5 (6H)-yl) piperidine-2,6-dione was dissolved in 40 mL of THF, and 0.102 g of 10% Pd/C was added as catalyst. The mixture was allowed to react for 4 hours under a stream of hydrogen. Then, hydrogen was replaced with argon and the catalyst was removed. The solvent was evaporated, and anhydrous ether was added to give 0.048 g of solid which was extremely sensitive to air. MS (*m*/*z*): 264 [M -1]⁺.

### Example 19

### 3-methyl-3-(1-nitro-4-oxo-4H-thiophene [3,4-c] pyrrole-5 (6H)-yl) piperidine-2,6-dione

3-methyl-3-(4-oxo-4H-thiophene [3,4-c] pyrrole-5 (6H)-yl) piperidine-2,6-dione was nitrated according to the method of Example 17 to give the title compound, MS (*m*/*z*): 308 [M-1]⁺.

### Example 20

### 3-methyl-3-(1-amino-4-oxo-4H-thiophene [3,4-c] pyrrole-5 (6H)-yl) piperidine-2,6-dione

3-methyl-3-(1-nitro-4-oxo-4H-thiophene [3,4-c] pyrrole-5 (6H)-yl) piperidine-2,6-dione was deoxidized according to the method of Example 18 to give the title compound. MS (*m*/*z*): 278 [M-1]⁺.

### Example 21

### 5-(4-oxo-4H-thiophene [3,4-c] pyrrole-5 (6H)-yl) piperidine-2-one

The title compound was prepared from 5-amino-piperidine-2-one following the method of Example 9. MS (*m*/*z*):235 [M-1]⁻.

### Example 22

### 3-(4-oxo-4H-thiophene [3,4-c] pyrrole-5 (6H)-yl) piperidine-2-one

The title compound was prepared from 3-amino-piperidine-2-one following the method of Example 9. MS (*m*/*z*): 235 [M-1]⁻

### Example 23

### 1-(2-hydroxyethyl)-3-(4-oxo-4H-thiophene [3,4-c] pyrrole-5 (6H)-yl) piperidine-2,6-dione

Following Example 10 and substituting iodomethane with 2-bromo-ethyl alcohol, a white solid was obtained. MS (*m*/*z*): 295 [M+1]⁺

### Example 24

### 2-(2,6-dioxo-3-(4-oxo-4H-thiophene [3,4-c] pyrrole-5 (6H)-yl) piperidine-1-yl) ethyl bromo acetate

138.95 mg of bromo-acetic acid and 280 mg of 1-(2-hydroxy -ethyl)-3-(4-oxo-4H-thiophene [3,4-c] pyrrole-5 (6H)-yl) piperidine-2,6-dione were dissolved in 20 mL of DCM. The mixture was stirred at room temperature with an electromagnetic stirrer. 206 mg of DCC was added and the mixture was allowed to stand overnight. Then, cyclohexyl urea was removed by filtration, and the filter cake was washed with DCM several times. The filtrates were combined, washed with saturated sodium chloride aqueous solution three times (30 mL each time), dried over anhydrous MgSO₄, filtered to remove drying medium, and evaporated to remove the solvent and obtain 254 mg of a white solid.

### Example 25

### 2-(2,6-dioxo-3-(4-oxo-4H-thiophene [3,4-c] pyrrole-5 (6H)-yl) piperidine-1-yl) ethyl diethylamino acetate

200 mg of 2-(2,6-dioxo-3-(4-oxo-4H-thiophene [3,4-c] pyrrole-5 (6H)-yl) piperidine-1-yl) ethyl bromo acetate was dissolved in 10 mL of DMF, and 800 mg of K₂CO₃ powder was added. Then 0.3 mL of diethylamine solution was added dropwise, and the reaction mixture was stirred at room temperature for 24 hours. The solvent and residual diethylamine were removed by vacuum distillation. The remaining residue was purified by silica gel column (mobile phase: ethyl acetate: petroleum ether = 2:1) to give 109 mg of white solid. MS (m/e): 408 (M+H⁺).

### Example 26

### 2-(2,6-dioxo-3-(4-oxo-4H-thiophene [3,4-c] pyrrole-5 (6H)-yl) piperidine-1-yl) ethyl (S)-Boc-proline ester

37 mg of (S)-Boc-carbonyl proline and 50 mg of 1-(2-hydroxy ethyl)-3-(4-oxo-4H-thiophene [3, 4-c] pyrrole-5 (6H)-yl) piperidine-2,6-dione were dissolved in 5 mL of DCM. The mixture was stirred with electromagnetic stirring at room temperature. 35 mg of DCC and 3 mg of DMAP were added, and the mixture was allowed to stand overnight. Cyclohexyl urea was removed by filtration, and the filter cake was washed with DCM several times. The filtrates were combined, dried over anhydrous MgSO₄, and evaporated to remove the solvent to give a crude product. The product was purified by column chromatography (stationary phase was silica gel, mobile phase was chloroform: acetone = 9:2) to give 58 mg of white solid.

### Example 27

### 2-(2,6-dioxo-3-(4-oxo-4H-thiophene [3,4-c] pyrrole-5 (6H)-yl) piperidine-1-yl) ethyl (S)-proline ester

48 mg of the title compound of Example 26 was dissolved in 10 mL of 25% TFA/DCM solution. The mixture was stirred for 4 hours with electromagnetic stirring. DCM and most of the TFA were evaporated to give a foam. The foam was dissolved in 50 mL of DCM, washed with saturated NaHCO₃ and NaCl aq. solution, and dried over anhydrous MgSO₄. Solvent was removed in vacuo and residual solvent was removed using high vacuum pump to give 38 mg of solid product. MS (*m*/*z*): 408 [M+1]⁺.

The compounds of Examples 28, 29 and 30 were prepared following the method of Example 27.

### Example 28

### 2-(2,6-dioxo-3-(4-oxo-4H-thiophene [3, 4-c] pyrrole-5 (6H)-yl) piperidine-1-yl) ethyl (S)-alanine ester

### Example 29

### 2-(2,6-dioxo-3-(4-oxo-4H-thiophene [3, 4-c] pyrrole-5 (6H)-yl) piperidine-1-yl) ethyl (S)-valine ester

### Example 30

### 2-(2,6-dioxo-3-(4-oxo-4H-thiophene [3, 4-c] pyrrole-5 (6H)-yl) piperidine-1-yl) ethyl (S)-phenylalanine ester

### Example 31

### 2-(2,6-dioxo-3-(4-oxo-4H-thiophene [3, 4-c] pyrrole-5 (6H)-yl) piperidine-1-yl) ethyl nicotinate

60 mg of nicotinoyl chloride hydrochloride and 40 mg of 1-(2-hydroxy ethyl)-3-(4-oxo-4H-thiophene [3, 4-c] pyrrole-5 (6H)-yl) piperidine-2,6-dione were dissolved in 10 mL of DCM. The mixture was stirred with electromagnetic stirring at room temperature. Then 100 mL of triethylamine and 3 mg of DMAP were added to the mixture, and the mixture was allowed to stand overnight. The mixture was then washed with NaHCO₃ and saturated NaCl, dried over anhydrous MgSO₄, and evaporated to remove the solvent to give a crude product. The product was purified by column chromatography (stationary phase was silica gel, mobile phase was chloroform: acetone = 9:2) to give 46 mg of white solid. MS (*m*/*z*): 416 [M+1]⁺.

### Example 32

### Tert-butyl 3-(bromomethyl)thiophene-2-carboxylate

To a 100 mL three-necked flask, equipped with an electromagnetic stirrer, and a reflux condenser (with an inert gas duct), 3.28 g of tert-butyl 3-methyl thiophene-2-carboxylate and 1.86 g of NBS were dissolved in 80 mL of dry CCl₄. 0.1 g of benzoyl peroxide was added. The mixture was refluxed for an hour, then cooled, and 50 mL of ethyl acetate was added. The organic layer was washed successively with 5% NaCO₃ aqueous solution twice, water and saturated brine once, dried over anhydrous MgSO₄, filtered, evaporated to remove the solvent, and purified by column chromatography to give 3.14 g of oil-like product.

### Example 33

### 3-(6-oxo-4H-thiophene [3,2-c] pyrrole-5 (6H)-yl) piperidine-2,6-dione

The title compound was prepared from tert-butyl 3-bromomethyl thiophene-2-carboxylate following the method of Example 9, MS (*m*/*z*): 249 [M -1]⁺.

### Example 34

### 5-(6-oxo-piperidine-3-yl)-5H-thiophene [3, 4-c] pyrrole-4,6-dione

1.3 g of thiophene [3,4-c] furan-1, 3-dione and 1.14 g of 5-amino -piperidine-2-one were dissolved in glacial acetic acid, refluxed for 6 hours. The acetic acid was removed in vacuo, and the resultant solid was dissolved in 60 mL of dry THF.1.8 g of CDI was added, and the reaction mixture was refluxed for 4 hours. The mixture was then cooled to 5°C, and allowed to stand for 4 hours, then filtered. The filter cake was washed with THF twice, and vacuum dried overnight to give 1.65 g of a white solid, MS (*m*/*z*): 249 [M-1]⁺.

While particular embodiments of the invention have been shown and described, it will be obvious to those skilled in the art that changes and modifications may be made without departing from the invention in its broader aspects, and therefore, the aim in the appended claims is to cover all such changes and modifications as fall within the true spirit and scope of the invention.

## Claims

1. A compound of Formula (**I**) or Formula (**II**), a pharmaceutically acceptable salt, or a hydrate thereof, 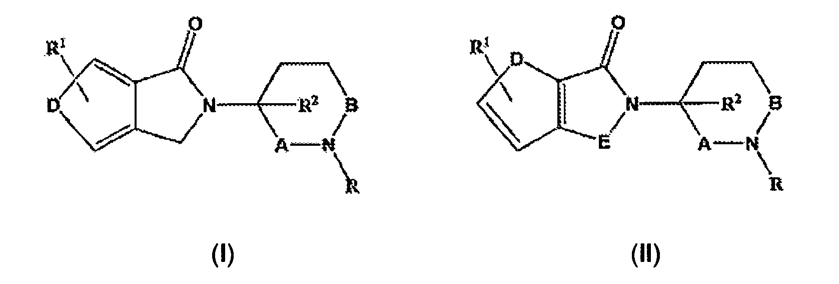 wherein
A, B and E independently represent CH₂, or CO;
D represents S, NH, or NC₁₋₆ alkylhydrocarbyl;
R represents H or R³;
R¹ represents H, or one or two same or different occurrences of F, Cl, Br, C₁₋₄alkylhydrocarbyl, OH, OC₁₋₄alkylhydrocarbyl, NO₂, NHC(O)C₁₋₄alkylhydrocarbyl, NH₂, NH(C₁₋₄alkylhydrocarbyl), or N(C_{1 -4}alkylhydrocarbyl)₂;
R² at each occurrence represents F, CF₃, H, or C_{1 -4}alkylhydrocarbyl;
R³ at each occurrence represents (CH₂)ₘR⁴, and m represents an integer from 1 to 6;
R⁴ represents F, Cl, H, C₁₋₄alkylhydrocarbyl, OH, OC_{1 -4}alkylhydrocarbyl, NHC(O)C₁₋₄alkylhydrocarbyl, NH₂, NH(C_{1 -4}alkylhydrocarbyl), N(C₁₋₄alkylhydrocarbyl)₂, or O₂CR⁵;
R⁵ represents CHR⁶NR⁷R⁸, CHR⁶NR⁹C(O)CHR¹⁰NR⁷R⁸, heterocycle W, or CHR⁶NR⁹C(O)W;
R⁶, R⁹ and R¹⁰ independently represent H or C_{1 -4}alkylhydrocarbyl;
R⁷ and R⁸ independently represent H, C₁₋₄alkylhydrocarbyl, or taken together in combination represent 1,3-propylidene, 1,4-butylene, 1,5-pentylene, 1,6-hexylidene, and
W represents a saturated or an unsaturated four to eight -membered heterocycle.

2. The compound of claim 1, wherein m is 1, 2 or 3.

3. The compound of claim 1, wherein R¹ represents H, F, NO₂, NH₂, NHCH₃, N(CH₃)₂, NHCOCH₃, OH, or OCH₃.

4. The compound of claim 1, wherein R² represents H, F, or CH₃.

5. The compound of claim 1, wherein
R represents H, CH₃, CH₂CH₃, CH₂CH₂CH₃, CH(CH₃)₂, CH₂CH₂CH₂CH₃, CH₂CH(CH₃)₂, CH(CH₃)CH₂CH₃, HOCH₂, HOCH₂CH₂, HOCH₂CH₂CH₂, MeOCH₂, MeOCH₂CH₂, MeOCH₂CH₂CH₂, EtOCH₂, EtOCH₂CH₂, EtOCH₂CH₂CH₂, (CH₂)_{1 -3}O₂CCHR⁶NR⁷R⁸,
R⁶ represents H or C₁₋₄alkylhydrocarbyl;
R⁷ and R⁸ independently represent H, or C₁₋₄alkylhydrocarbyl, or taken together in combination represent 1,3-propylidene, 1,4-butylene, 1,5-pentylene, 1,6-hexylidene, or (CH₂)₁₋₃O₂CW, and
W represents 2-pyridyl, 3-pyridyl, 4-pyridyl, pyrrolidine, or substituted pyrrolidine.

6. Use of a compound of Formula (**I**) or Formula (**II**), a pharmaceutically acceptable salt, or hydrate thereof in the preparation of a medicine, 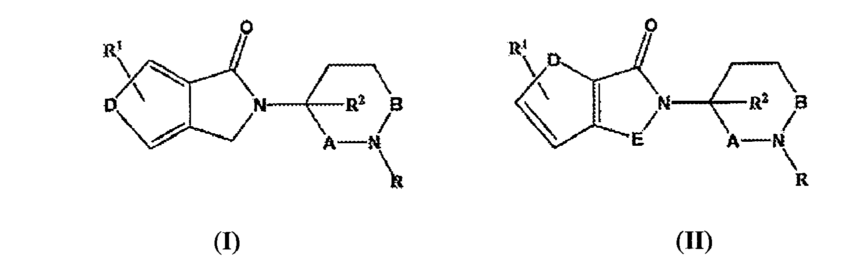 wherein
A, B and E independently represent CH₂ or CO;
D represents S, NH, or NC₁₋₆ alkylhydrocarbyl;
R represents H, C₁₋₆alkylhydrocarbyl, or R³;
R¹ represents H, or one or two same or different occurrences of F, Cl, Br, C₁₋₄alkylhydrocarbyl, OH, OC₁₋₄alkylhydrocarbyl, NO₂, NHC(O)C₁₋₄alkylhydrocarbyl, NH₂, NH(C₁₋₄alkylhydrocarbyl), or N(C_{1 -4}alkylhydrocarbyl)₂;
R² at each occurrence represents F, CF₃, H, or C_{1 -4}alkylhydrocarbyl;
R³ at each occurrence represents (CH₂)ₘR⁴, and m represents an integer from 1 to 6;
R⁴ represents F, Cl, H, C₁₋₄alkylhydrocarbyl, OH, OC_{1 -4}alkylhydrocarbyl, NHC(O)C₁₋₄alkylhydrocarbyl, NH₂, NH(C_{1 -4}alkylhydrocarbyl), N(C₁₋₄alkylhydrocarbyl)₂, or O₂CR⁵;
R⁵ represents CHR⁶NR⁷R⁸, CHR⁶NR⁹C(O)CHR¹⁰NR⁷R⁸, heterocycle W, or CHR⁶NR⁹C(O)W;
R⁶, R⁹ and R¹⁰ independently represent H or C_{1 -4}alkylhydrocarbyl;
R⁷ and R⁸ independently represents H, C₁₋₄alkylhydrocarbyl, or taken together in combination represent 1,3-propylidene, 1,4-butylene, 1,5-pentylene, 1,6-hexylidene, and
W represents a saturated or an unsaturated four to eight -membered heterocycle;
wherein
the medicine can alleviate or treat diseases or physiological disorders.

7. A pharmaceutical composition comprising at least a compound of
Formula (I) or Formula (II), 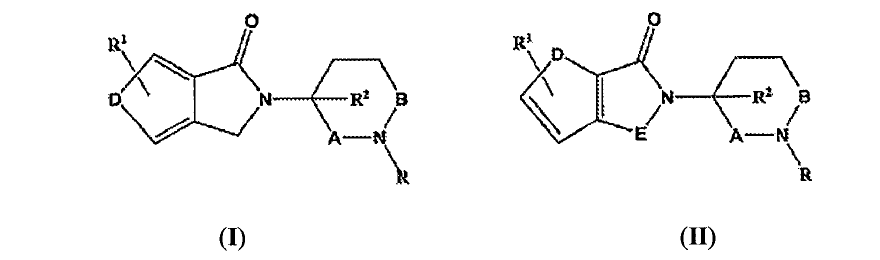 wherein
A, B and E independently represent CH₂ or CO;
D represents S, NH, or NC₁₋₆ alkylhydrocarbyl;
R represents H, C₁₋₆alkylhydrocarbyl, or R³;
R¹ represents H, or one or two same or different occurrences of F, Cl, Br, C₁₋₄alkylhydrocarbyl, OH, OC₁₋₄alkylhydrocarbyl, NO₂, NHC(O)C₁₋₄alkylhydrocarbyl, NH₂, NH(C₁₋₄alkylhydrocarbyl), or N(C_{1 -4}alkylhydrocarbyl)₂;
R² at each occurrence represents F, CF₃, H, or C_{1 -4}alkylhydrocarbyl;
R³ at each occurrence represents (CH₂)ₘR⁴, and m represents an integer from 1 to 6;
R⁴ represents F, Cl, H, C₁₋₄alkylhydrocarbyl, OH, OC_{1 -4}alkylhydrocarbyl, NHC(O)C₁₋₄alkylhydrocarbyl, NH₂, NH(C₁ -₄alkylhydrocarbyl), N(C₁₋₄alkylhydrocarbyl)₂ or O₂CR⁵;
R⁵ represents CHR⁶NR⁷R⁸, CHR⁶NR⁹C(O)CHR¹⁰NR⁷R⁸, heterocycle W or CHR⁶NR⁹C(O)W;
R⁶, R⁹ and R¹⁰ independently represent H or C_{1 -4}alkylhydrocarbyl;
R⁷ and R⁸ independently represent H or C₁₋₄alkylhydrocarbyl, or taken together in combination represent 1,3-propylidene, 1,4-butylene, 1,5-pentylene, 1,6-hexylidene, and
W represents a saturated or an unsaturated four to eight -membered heterocycle;
wherein
the pharmaceutical composition can alleviate or treat diseases or physiological disorders.

8. The use or composition of claims 6 or 7, wherein the diseases or physiological disorders are inflammatory diseases or infectious diseases.

9. The use or composition of claims 6 or 7, wherein the diseases or physiological disorders are diseases of the immune system.

10. The use or composition of claims 6 or 7, wherein the diseases or physiological disorders are malignant tumors.

11. The use or composition of claims 6 or 7, wherein the diseases or physiological disorders are: septic shock, endotoxic shock, hemodynamic shock, septic syndrome, post ischemic reperfusion injury, malaria, mycobacterial infection, meningitis, psoriasis, congestive heart failure, fibrotic disease, cachexia, transplant immune rejection, cancer, autoimmune disease, opportunistic infection in AIDS, erythema nodosum leprosy, lupus erythematosus, refractory lupus erythematosus, Behcet syndrome, regional ileitis, myelodysplastic syndrome, rheumatoid arthritis (RA), hepatitis, nephritis, rheumatoid spondylitis, multiple myeloma, melanoma, thyroid tumor, kidney cancer, prostate cancer, lymphoma, leukemia, liver cancer, brain glioma, colorectal cancer, lung cancer, stomach cancer, or breast cancer.

12. A method of preparing a compound of Formula (I) or Formula (II), 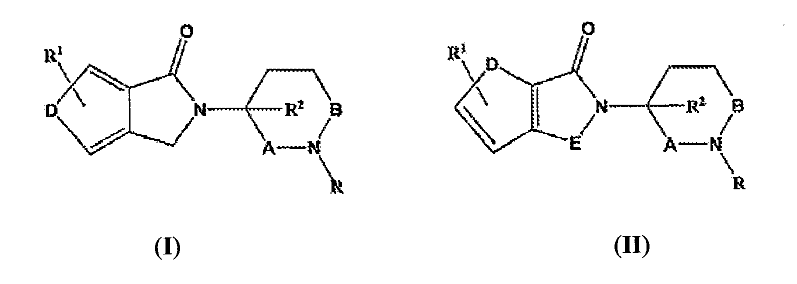 wherein
A, B and E independently represent CH₂ or CO;
D represents S, NH, or NC₁₋₆ alkylhydrocarbyl;
R represents H, C₁₋₆alkylhydrocarbyl, or R³;
R¹ represents H, or one or two same or different occurrences of F, Cl, Br, C₁₋₄alkylhydrocarbyl, OH, OC₁₋₄alkylhydrocarbyl, NO₂, NHC(O)C₁₋₄alkylhydrocarbyl, NH₂, NH(C₁₋₄alkylhydrocarbyl), or N(C_{1 -4}alkylhydrocarbyl)2;
R² at each occurrence represents F, CF₃, H, or C_{1 -4}alkylhydrocarbyl;
R³ at each occurrence represents (CH₂)ₘR⁴, and m represents an integer from 1 to 6;
R⁴ represents F, Cl, H, C₁₋₄alkylhydrocarbyl, OH, OC_{1 -4}alkylhydrocarbyl, NHC(O)C₁₋₄alkylhydrocarbyl, NH₂, NH(C_{1 -4}alkylhydrocarbyl), N(C₁₋₄alkylhydrocarbyl)₂, or O₂CR⁵;
R⁵ represents CHR⁶NR⁷R⁸, CHR⁶NR⁹C(O)CHR¹⁰NR⁷R⁸, heterocycle W or CHR⁶NR⁹C(O)W;
R⁶, R⁹ and R¹⁰ independently represent H or C_{1 -4}alkylhydrocarbyl;
R⁷ and R⁸ independently represent H or C₁₋₄alkylhydrocarbyl, or taken together in combination represent 1,3-propylidene, 1,4-butylene, 1,5-pentylene, or 1,6-hexylidene, and
W represents a saturated or an unsaturated four to eight -membered heterocycle;
wherein the method comprises the steps of:
(1) contacting a compound of Formula **(VII),** or Formula **(VIII)** with a compound of Formula **(X)** to obtain a compound of Formula **(XI)** or Formula **(XII),** 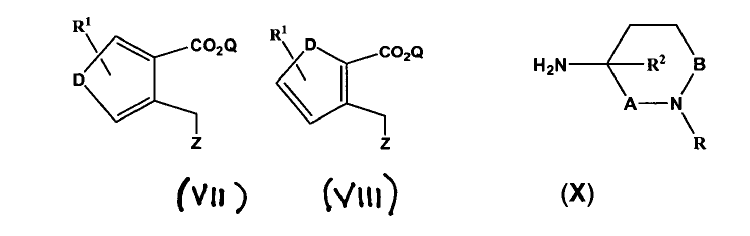 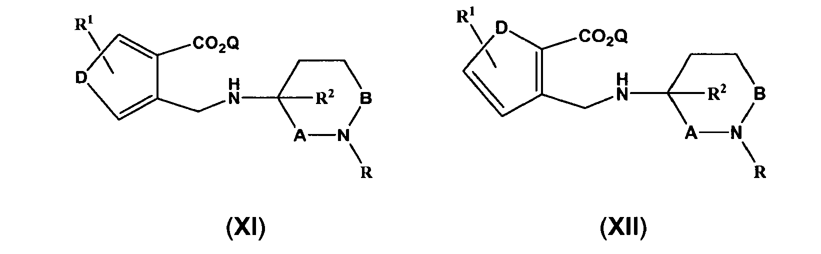 wherein the definitions of A, B, D, R, R¹, R² are the same as that for Formula **(I)** or Formula **(II),** Z represents Cl, Br, I, Ms, Ts, and Q represents methyl, tert-butyl;
(2) hydrolyzing the compound of Formula **(XI)** or Formula **(XII)** to obtain a corresponding acid of Formula **(XIII)** or Formula (**XIV**) 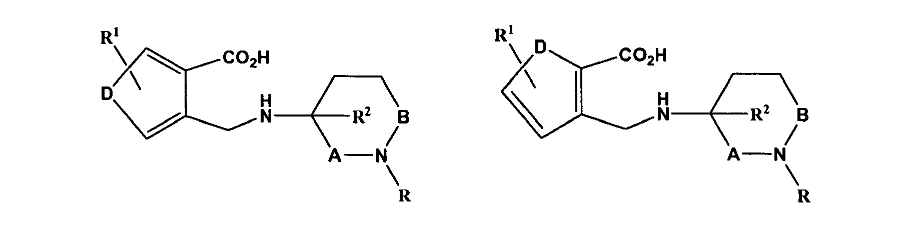 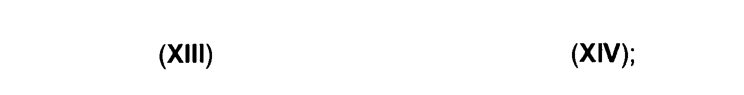 and
(3) dehydrating and cyclizing the compound of Formula **(XIII)** or Formula **(XIV)** to obtain the compound of Formula **(I)** or Formula **(II).**

13. A method of preparing a compound of Formula **(I)** or Formula **(II)** comprising contacting a compound of Formula **(XV)** or Formula **(XVI)** with a compound of Formula L-R to obtain the compound of Formula **(I)** or Formula **(II),** wherein the definition of A, B, D, E, R, R¹, R² are the same as that for Formula **(I)** or Formula **(II),** L represents Cl, Br, I, Ms or Ts; R is the same as that for Formula **(I)** or Formula **(II).**

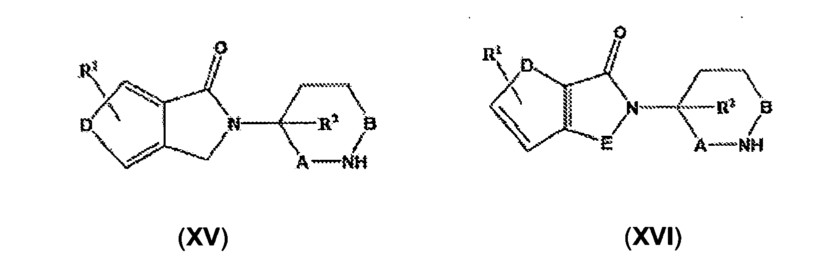

14. The pharmaceutical composition of claim 7, further comprising a pharmaceutically acceptable carrier, excipient, filler, solvent, diluent, coloring agent, or adhesive, wherein the administration mode of the compound of Formula **(I)** or Formula **(II)** is gastrointestinal tract administration, oral administration, intravenous injection, intraperitoneal injection, dermal injection, intramuscular injection, intranasal administration, intraocular administration, administration by inhalation, rectal administration, reproductive tract administration, or percutaneous absorption.

## Patentansprüche

1. Verbindung der Formel **(I)** oder Formel **(II),** pharmazeutisch akzeptables Salz oder Hydrat davon, 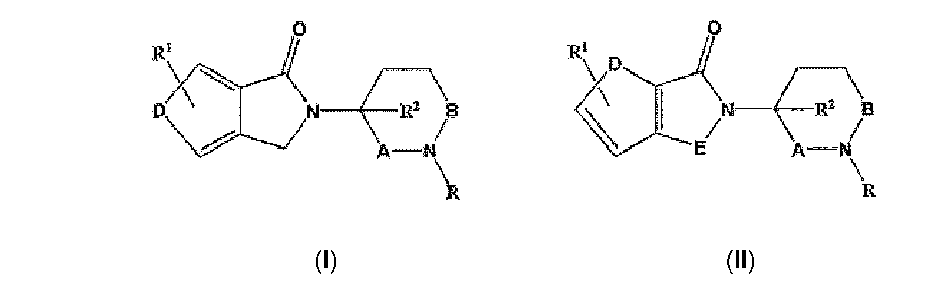 wobei
A, B und E unabhängig CH₂ oder CO darstellen;
D S, NH oder NC₁₋₆-Alkylhydrocarbyl darstellt;
R H oder R³ darstellt;
R¹ H oder ein oder zwei gleiche oder verschiedene Vorkommnisse von F, Cl, Br, C₁₋₄-Alkylhydrocarbyl, OH, OC_{1 -4}-Alkylhydrocarbyl, NO₂, NHC(O)C₁₋₄-Alkylhydrocarbyl, NH₂, NH(C_{1 -4}-Alkylhydrocarbyl) oder N(C₁₋₄-Alkylhydrocarbyl)₂ darstellt;
R² bei jeder Vorkommnis F, CF₃, H oder C₁₋₄-Alkylhydrocarbyl darstellt;
R³ bei jeder Vorkommnis (CH₂)ₘR⁴ darstellt und m eine ganze Zahl von 1 to 6 darstellt;
R⁴ F, Cl, H, C₁₋₄-Alkylhydrocarbyl, OH, OC₁₋₄-Alkylhydrocarbyl, NHC(O)C₁₋₄-Alkylhydrocarbyl, NH₂, NH(C₁₋₄-Alkylhydrocarbyl), N(C_{1 -4}-Alkylhydrocarbyl)₂ oder O₂CR⁵ darstellt;
R⁵ CHR⁶NR⁷R⁸, CHR⁶NR⁹C(O)CHR¹⁰NR⁷R⁸, heterocyclisches W oder CHR⁶NR⁹C(O)W darstellt;
R⁶, R⁹ und R¹⁰ unabhängig H oder C₁₋₄-Alkylhydrocarbyl darstellen;
R⁷ und R⁸ unabhängig H, C₁₋₄-Alkylhydrocarbyl darstellen oder zusammengenommen in Kombination 1,3-Propyliden, 1,4- Butylen, 1,5 -Pentylen, 1,6-Hexyliden darstellen und
W einen gesättigte oder einen ungesättigten vier- bis achtgliedrigen Heterocyclus darstellt.

2. Verbindung nach Anspruch 1, wobei m 1, 2 oder 3 beträgt.

3. Verbindung nach Anspruch 1, wobei R¹ H, F, NO₂, NH₂, NHCH₃, N(CH₃)₂, NHCOCH₃, OH oder OCH₃ darstellt.

4. Verbindung nach Anspruch 1, wobei R² H, F oder CH₃ darstellt.

5. Verbindung nach Anspruch 1, wobei
R H, CH₃, CH₂CH₃, CH₂CH₂CH₃, CH(CH₃)₂, CH₂CH₂CH₂CH₃, CH₂CH(CH₃)₂, CH(CH₃)CH₂CH₃, HOCH₂, HOCH₂CH₂, HOCH₂CH₂CH₂, MeOCH₂, MeOCH₂CH₂, MeOCH₂CH₂CH₂, EtOCH₂, EtOCH₂CH₂, EtOCH₂CH₂CH₂, (CH₂)₁₋₃O₂CCHR⁶NR⁷R⁸ darstellt,
R⁶ H oder C₁₋₄-Alkylhydrocarbyl darstellt;
R⁷ und R⁸ unabhängig H oder C₁₋₄-Alkylhydrocarbyl darstellen oder zusammengenommen in Kombination 1,3-Propyliden, 1,4-Butylen, 1,5-Pentylen, 1,6-Hexyliden oder (CH₂)₁₋₃O₂CW darstellen und
W 2-Pyridyl, 3-Pyridyl, 4-Pyridyl, Pyrrolidin oder substituiertes Pyrrolidin darstellt.

6. Verwendung einer Verbindung der Formel **(I)** oder Formel **(II),** eines pharmazeutisch akzeptablen Salzes oder Hydrats davon bei der Herstellung eines Medikaments, 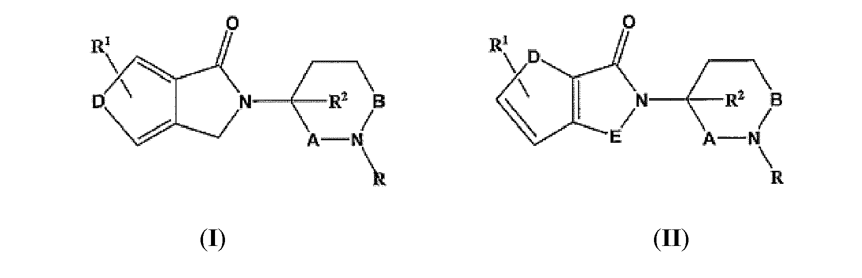 wobei
A, B und E unabhängig CH₂ oder CO darstellen;
D S, NH oder NC₁₋₆-Alkylhydrocarbyl darstellt;
R H, C₁₋₆-Alkylhydrocarbyl oder R³ darstellt;
R¹ H oder ein oder zwei gleiche oder verschiedene Vorkommnisse von F, Cl, Br, C₁₋₄-Alkylhydrocarbyl, OH, OC_{1 -4}-Alkylhydrocarbyl, NO₂, NHC(O)C₁₋₄-Alkylhydrocarbyl, NH₂, NH(C_{1 -4}-Alkylhydrocarbyl) oder N(C₁₋₄-Alkylhydrocarbyl)₂ darstellt;
R² bei jedem Vorkommnis F, CF₃, H oder C₁₋₄-Alkylhydrocarbyl darstellt;
R³ bei jeder Vorkommnis (CH₂)ₘR⁴ darstellt und m eine ganze Zahl von 1 to 6 darstellt;
R⁴ F, Cl, H, C₁₋₄-Alkylhydrocarbyl, OH, OC₁₋₄-Alkylhydrocarbyl, NHC(O)C₁₋₄-Alkylhydrocarbyl, NH₂, NH(C₁₋₄-Alkylhydrocarbyl), N(C_{1 -4}-Alkylhydrocarbyl)₂ oder O₂CR⁵ darstellt;
R⁵ CHR⁶NR⁷R⁸, CHR⁶NR⁹C(O)CHR¹⁰NR⁷R⁸, heterocyclisches W oder CHR⁶NR⁹C(O)W darstellt;
R⁶, R⁹ und R¹⁰ unabhängig H oder C₁₋₄-Alkylhydrocarbyl darstellen;
R⁷ und R⁸ unabhängig H, C₁₋₄-Alkylhydrocarbyl darstellen oder zusammengenommen in Kombination 1,3-Propyliden, 1,4-Butylen, 1,5 -Pentylen, 1,6-Hexyliden darstellen und
W einen gesättigten oder ungesättigten vier bis achtgliedrigen Heterocyclus darstellt;
wobei
das Medikament Krankheiten oder physiologische Beschwerden mildern oder behandeln kann.

7. A pharmazeutische Zusammensetzung umfassend mindestens eine
Verbindung der Formel (**I**) oder Formel (**II**), 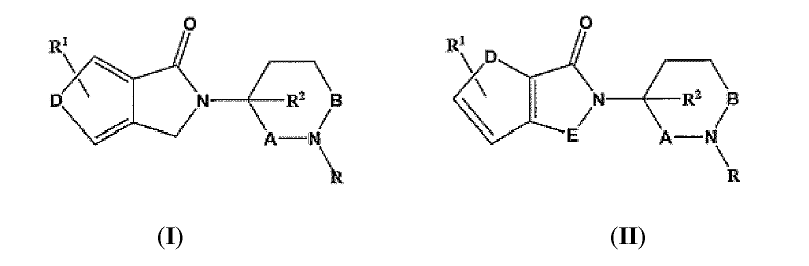 wobei
A, B und E unabhängig CH₂ oder CO darstellen;
D S, NH oder NC₁₋₆-Alkylhydrocarbyl darstellt;
R H, C₁₋₆-Alkylhydrocarbyl oder R³ darstellt;
R¹ H oder eine oder zwei gleiche oder verschiedene Vorkommnisse von F, Cl, Br, C₁₋₄-Alkylhydrocarbyl, OH, OC_{1 -4}-Alkylhydrocarbyl, NO₂, NHC(O)C₁₋₄-Alkylhydrocarbyl, NH₂, NH(C_{1 -4}-Alkylhydrocarbyl) oder N(C₁₋₄-Alkylhydrocarbyl)₂ darstellt;
R² bei jeder Vorkommnis F, CF₃, H oder C₁₋₄-Alkylhydrocarbyl darstellt;
R³ bei jeder Vorkommnis (CH₂)ₘR⁴ darstellt und m eine ganze Zahl von 1 to 6 darstellt;
R⁴ F, Cl, H, C₁₋₄-Alkylhydrocarbyl, OH, OC₁₋₄-Alkylhydrocarbyl, NHC(O)C₁₋₄-Alkylhydrocarbyl, NH₂, NH(C₁₋₄-Alkylhydrocarbyl), N(C_{1 -4}-Alkylhydrocarbyl)₂ oder O₂CR⁵ darstellt;
R⁵ CHR⁶NR⁷R⁸, CHR⁶NR⁹C(O)CHR¹⁰NR⁷R⁸, heterocyclisches W oder CHR⁶NR⁹C(O)W darstellt;
R⁶, R⁹ und R¹⁰ unabhängig H oder C₁₋₄-Alkylhydrocarbyl darstellen;
R⁷ und R⁸ unabhängig H oder C₁₋₄-Alkylhydrocarbyl darstellen oder zusammengenommen in Kombination 1,3-Propyliden, 1,4-Butylen, 1,5-Pentylen, 1,6-Hexyliden darstellen und
W einen gesättigten oder einen ungesättigten vier- bis achtgliedrigen Heterocyclus darstellt;
wobei
die pharmazeutische Zusammensetzung Krankheiten oder physiologische Beschwerden mildern oder behandeln kann.

8. Verwendung oder Zusammensetzung nach den Ansprüchen 6 oder 7, wobei die Krankheiten oder physiologischen Beschwerden entzündliche Krankheiten oder ansteckende Krankheiten sind.

9. Verwendung oder Zusammensetzung nach den Ansprüchen 6 oder 7, wobei die Krankheiten oder physiologischen Beschwerden Krankheiten des Immunsystems sind.

10. Verwendung oder Zusammensetzung nach den Ansprüchen 6 oder 7, wobei die Krankheiten oder physiologischen Beschwerden bösartige Tumore sind.

11. Verwendung von Zusammensetzungen nach den Ansprüchen 6 oder 7, wobei die Krankheiten oder physiologischen Beschwerden Folgendes sind: septischer Schock, endotoxischer Schock, hämodynamischer Schock, septisches Syndrom, postischämische Reperfusionsverletzung, Malaria, mycobakterielle Infektion, Meningitis, Schuppenflechte, dekompensierte Herzinsuffizienz, Fibrosekrankheit, Kachexie, Transplantationsimmunabstoßung, Krebs, Autoimmunkrankheit, opportunistische Infektion bei AIDS, Erythema nodosum leprosum, Lupus erythematodes, refraktärer Lupus erythematodes, Behcet-Krankheit, Crohn-Krankheit, myelodysplastisches Syndrom, rheumatoide Arthritis (RA), Hepatitis, Nephritis, Morbus Bechterew, multiples Myelom, Melanom, Schilddrüsentumor, Nierenkrebs, Prostatakrebs, Lymphom, Leukämie, Leberkrebs, Gehirngliom, Kolorektalkrebs, Lungenkrebs, Magenkrebs oder Brustkrebs.

12. Verfahren zum Herstellen der Formel (**I**) oder Formel (**II**), 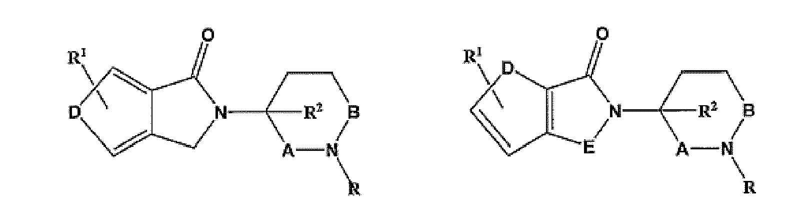 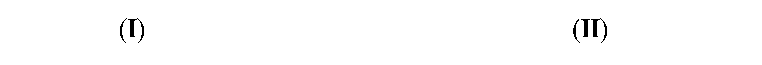 wobei
A, B und E unabhängig CH₂ oder CO darstellen;
D S, NH oder NC₁₋₆-Alkylhydrocarbyl darstellt;
R H, C₁₋₆-Alkylhydrocarbyl oder R³ darstellt;
R¹ H oder eine oder zwei gleiche oder verschiedene Vorkommnisse von F, Cl, Br, C₁₋₄-Alkylhydrocarbyl, OH, OC_{1 -4}-Alkylhydrocarbyl, NO₂, NHC(O)C₁₋₄-Alkylhydrocarbyl, NH₂, NH(C_{1 -4}-Alkylhydrocarbyl) oder N(C₁-₄-Alkylhydrocarbyl)₂ darstellt;
R² bei jeder Vorkommnis F, CF₃, H oder C₁₋₄-Alkylhydrocarbyl darstellt;
R³ bei jeder Vorkommnis (CH₂)ₘR⁴ darstellt und m eine ganze Zahl von 1 to 6 darstellt;
R⁴ F, Cl, H, C₁₋₄-Alkylhydrocarbyl, OH, OC₁₋₄-Alkylhydrocarbyl, NHC(O)C₁₋₄-Alkylhydrocarbyl, NH₂, NH(C₁₋₄-Alkylhydrocarbyl), N(C_{1 -4}-Alkylhydrocarbyl)₂ oder O₂CR⁵ darstellt;
R⁵ CHR⁶NR⁷R⁸, CHR⁶NR⁹C(O)CHR¹⁰NR⁷R⁸, heterocyclisches W oder CHR⁶NR⁹C(O)W darstellt;
R⁶, R⁹ und R¹⁰ unabhängig H oder C₁₋₄-Alkylhydrocarbyl darstellen;
R⁷ und R⁸ unabhängig H oder C₁₋₄-Alkylhydrocarbyl darstellen oder zusammengenommen in Kombination 1,3-Propyliden, 1,4-Butylen, 1,5-Pentylen oder 1,6-Hexyliden darstellen und
W einen gesättigten oder einen ungesättigten vier- bis achtgliedrigen Heterocyclus darstellt;
wobei das Verfahren die Schritte umfasst des:
(1) Kontaktierens der Verbindung der Formel **(VII)** oder Formel **(VIII)** mit einer Verbindung der Formel **(X),** um die Verbindung der Formel **(XI)** oder Formel **(XII),** 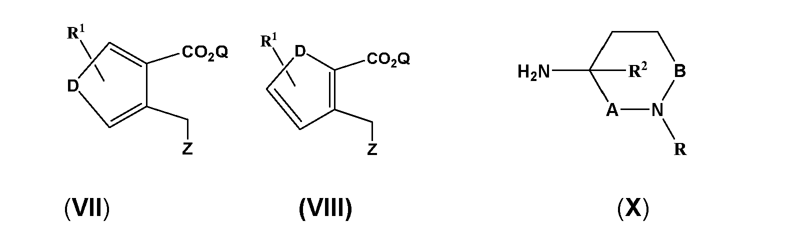 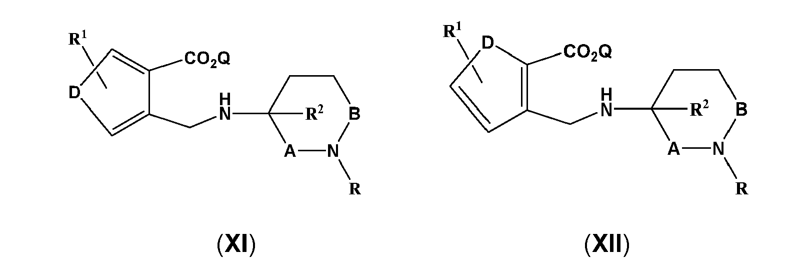 zu erhalten, wobei die Definitionen von A, B, D, R, R¹, R² gleich wie diejenige für die Formel **(I)** oder Formel **(II)** sind, Z Cl, Br, I, Ms, Ts darstellt und Q Methyl, tert-Butyl darstellt;
(2) Hydrolysierens der Verbindung der Formel **(XI)** oder Formel
**(XII),** um eine entsprechende Säure der Formel **(XIII)** oder Formel **(XIV)** 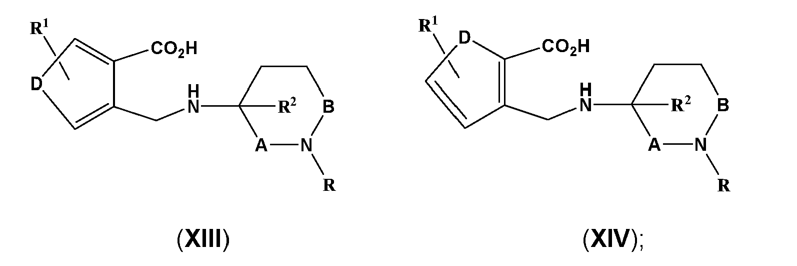 zu erhalten und
(3) Dehydratisierens und Cyclisierens der Verbindung der Formel **(XIII)** oder Formel **(XIV),** um die Verbindung der Formel **(I)** oder Formel **(II)** zu erhalten.

13. Verfahren zum Herstellen einer Verbindung der Formel **(I)** oder Formel **(II),** umfassend das Kontaktieren einer Verbindung der Formel **(XV)** oder Formel **(XVI)** mit einer Verbindung der Formel L-R, um die Verbindung der Formel (I) oder Formel **(II)** zu erhalten, wobei die Definitionen von A, B, D, E, R, R¹, R² gleich wie diejenigen für die Formel (I) oder Formel **(II)** sind, L Cl, Br, I, Ms oder Ts darstellt; R gleich wie dasjenige für die Formel (I) oder Formel **(II)** 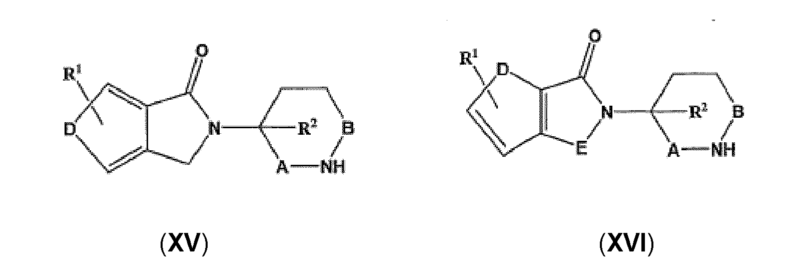 ist.

14. Pharmazeutische Zusammensetzung nach Anspruch 7, des Weiteren einen pharmazeutisch akzeptablen Träger, ein pharmazeutisch akzeptables Vehikel, einen pharmazeutisch akzeptablen Füllstoff, ein pharmazeutisch akzeptables Lösungsmittel, Verdünnungsmittel, Farbmittel, oder einen pharmazeutisch akzeptablen Klebstoff umfassend, wobei der Verabreichungsmodus der Verbindung der Formel (I) oder Formel **(II)** die Magen-Darm-Traktverabreichung, orale Verabreichung, intravenöse Injektion, intraperitoneale Injektion, dermale Injektion, intramuskuläre Injektion, intranasale Verabreichung, intraokuläre Verabreichung, Verabreichung durch Inhalation, rektale Verabreichung, Fortpflanzungstraktverabreichung oder perkutane Absorption ist.

## Revendications

1. Composé de formule **(I)** ou de formule **(II),** sel pharmaceutiquement acceptable de celui-ci, ou hydrate de celui-ci, 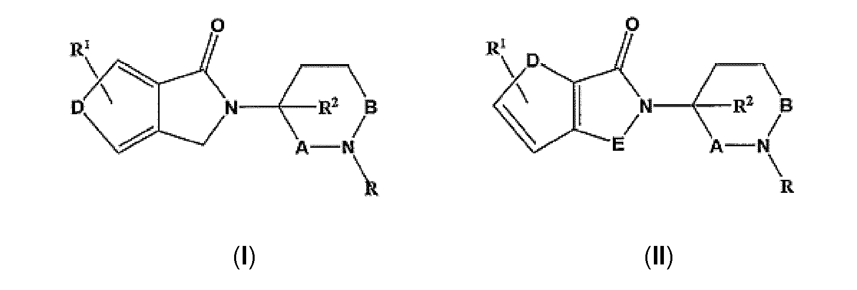 dans lesquelles
A, B et E représentent indépendamment CH₂, ou CO ;
D représente S, NH, ou N(alkyle en C₁₋₆)hydrocarbyle ;
R représente H ou R³ ;
R¹ représente H, ou un ou deux groupes identiques ou différents sont choisis parmi F, Cl, Br, (alkyle en C₁₋₄)hydrocarbyle, OH, O(alkyle en C₁₋₄)hydrocarbyle, NO₂, NHC(O)(alkyle en C₁₋₄)hydrocarbyle, NH₂, NH((alkyle en C₁₋₄)hydrocarbyle), ou N((alkyle en C₁₋₄)hydrocarbyle)₂ ;
R² dans chaque cas représente F, CF₃, H, ou (alkyle en C_{1 -4})hydrocarbyle ;
R³ dans chaque cas représente (CH₂)ₘR⁴, et m représente un nombre entier de 1 à 6 ;
R⁴ représente F, Cl, H, (alkyle en C₁₋₄)hydrocarbyle, OH, O(alkyle en C₁₋₄)hydrocarbyle, NHC(O)(alkyle en C₁₋₄)hydrocarbyle, NH₂, NH((alkyle en C₁₋₄)hydrocarbyle), N((alkyle en C₁₋₄)hydrocarbyle)₂, ou O₂CR⁵ ;
R⁵ représente CHR⁶NR⁷R⁸, CHR⁶NR⁹C(O)CHR¹⁰NR⁷R⁸, un hétérocycle W, ou CHR⁶NR⁹C(O)W ;
R⁶, R⁹ et R¹⁰ représentent indépendamment H ou (alkyle en C_{1 -4})hydrocarbyle ;
R^{7 et} R⁸ représentent indépendamment H, (alkyle en C_{1 -4})hydrocarbyle, ou pris ensemble en combinaison représentent un groupe 1,3-propylidène, 1,4-butylène, 1,5-pentylène, 1,6-hexylidène ; et W représente un hétérocycle saturé ou insaturé de quatre à huit éléments.

2. Composé selon la revendication 1, dans lequel m vaut 1, 2 ou 3.

3. Composé selon la revendication 1, dans lequel R¹ représente H, F, NO₂, NH₂, NHCH₃, N(CH₃)₂, NHCOCH₃, OH, ou OCH₃.

4. Composé selon la revendication 1, dans lequel R² représente H, F, ou CH₃.

5. Composé selon la revendication 1, dans lequel
R représente H, CH₃, CH₂CH₃, CH₂CH₂CH₃, CH(CH₃)₂, CH₂CH₂CH₂CH₃, CH₂CH(CH₃)₂, CH(CH₃)CH₂CH₃, HOCH₂, HOCH₂CH₂, HOCH₂CH₂CH₂, MeOCH₂, MeOCH₂CH₂, MeOCH₂CH₂CH₂, EtOCH₂, EtOCH₂CH₂, EtOCH₂CH₂CH₂, (CH2)₁₋₃O₂CCHR⁶NR⁷R⁸ ;
R⁶ représente H ou (alkyle en C₁₋₄)hydrocarbyle ;
R^{7 et} R⁸ représentent indépendamment H, ou (alkyle en C_{1 -4})hydrocarbyle, ou pris ensemble en combinaison représentent un groupe 1,3-propylidène, 1,4-butylène, 1,5-pentylène, 1,6-hexylidène, ou (CH₂)₁₋₃O₂CW; et
W représente un groupe 2-pyridyle, 3-pyridyle, 4-pyridyle, pyrrolidine, ou pyrrolidine substitué.

6. Utilisation d'un composé de formule (I) ou de formule (II), d'un sel pharmaceutiquement acceptable de celui-ci, ou d'un hydrate de celui-ci pour la préparation d'un médicament, 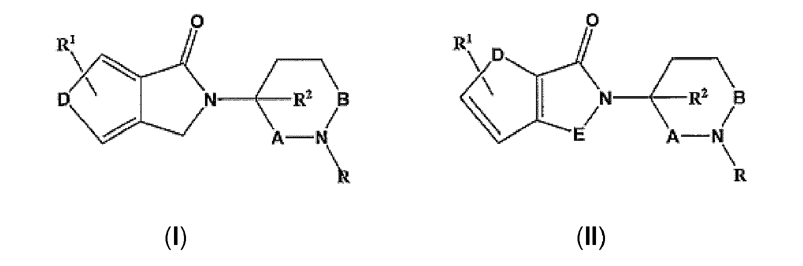 dans lesquelles
A, B et E représentent indépendamment CH₂, ou CO ;
D représente S, NH, ou N(alkyle en C₁₋₆)hydrocarbyle ;
R représente H ou R³ ;
R¹ représente H, ou un ou deux groupes identiques ou différents sont choisis parmi F, Cl, Br, (alkyle en C₁₋₄)hydrocarbyle, OH, O(alkyle en C₁₋₄)hydrocarbyle, NO₂, NHC(O)(alkyle en C₁₋₄)hydrocarbyle, NH₂, NH((alkyle en C₁₋₄)hydrocarbyle), ou N((alkyle en C₁₋₄)hydrocarbyle)₂ ;
R² dans chaque cas représente F, CF₃, H, ou (alkyle en C_{1 -4})hydrocarbyle ;
R³ dans chaque cas représente (CH₂)ₘR⁴, et m représente un nombre entier de 1 à 6 ;
R⁴ représente F, Cl, H, (alkyle en C₁₋₄)hydrocarbyle, OH, O(alkyle en C₁₋₄)hydrocarbyle, NHC(O)(alkyle en C₁₋₄)hydrocarbyle, NH₂, NH((alkyle en C₁₋₄)hydrocarbyle), N((alkyle en C₁₋₄)hydrocarbyle)₂, ou O₂CR⁵ ;
R⁵ représente CHR⁶NR⁷R⁸, CHR⁶NR⁹C(O)CHR¹⁰NR⁷R⁸, un hétérocycle W, ou CHR⁶NR⁹C(O)W ;
R⁶, R⁹ et R¹⁰ représentent indépendamment H ou (alkyle en C_{1 -4})hydrocarbyle ;
R^{7 et} R⁸ représentent indépendamment H, (alkyle en C_{1 -4})hydrocarbyle, ou pris ensemble en combinaison représentent un groupe 1,3-propylidène, 1,4-butylène, 1,5-pentylène, 1,6-hexylidène ; et W représente un hétérocycle saturé ou insaturé de quatre à huit éléments ;
le médicament pouvant soulager ou traiter des maladies ou des troubles physiologiques.

7. Composition pharmaceutique comprenant au moins un composé de
formule **(I)** ou de formule **(II),** 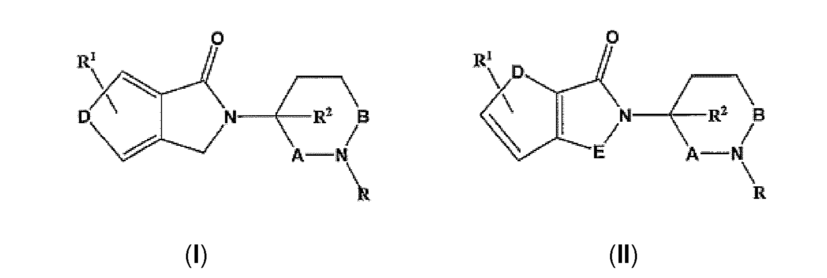 dans lesquelles
A, B et E représentent indépendamment CH₂, ou CO ;
D représente S, NH, ou N(alkyle en C₁₋₆)hydrocarbyle ;
R représente H ou R³ ;
R¹ représente H, ou un ou deux groupes identiques ou différents sont choisis parmi F, Cl, Br, (alkyle en C₁₋₄)hydrocarbyle, OH, O(alkyle en C₁₋₄)hydrocarbyle, NO₂, NHC(O)(alkyle en C₁₋₄)hydrocarbyle, NH₂, NH((alkyle en C₁₋₄)hydrocarbyle), ou N((alkyle en C₁₋₄)hydrocarbyle)₂ ;
R² dans chaque cas représente F, CF₃, H, ou (alkyle en C_{1 -4})hydrocarbyle ;
R³ dans chaque cas représente (CH₂)ₘR⁴, et m représente un nombre entier de 1 à 6 ;
R⁴ représente F, Cl, H, (alkyle en C₁₋₄)hydrocarbyle, OH, O(alkyle en C₁₋₄)hydrocarbyle, NHC(O)(alkyle en C₁₋₄)hydrocarbyle, NH₂, NH((alkyle en C₁₋₄)hydrocarbyle), N((alkyle en C₁₋₄)hydrocarbyle)₂, ou O₂CR⁵ ;
R⁵ représente CHR⁶NR⁷R⁸, CHR⁶NR⁹C(O)CHR¹⁰NR⁷R⁸, un hétérocycle W, ou CHR⁶NR⁹C(O)W ;
R⁶, R⁹ et R¹⁰ représentent indépendamment H ou (alkyle en C_{1 -4})hydrocarbyle ;
R^{7 et} R⁸ représentent indépendamment H, (alkyle en C_{1 -4})hydrocarbyle, ou pris ensemble en combinaison représentent un groupe 1,3-propylidène, 1,4-butylène, 1,5-pentylène, 1,6-hexylidène ; et W représente un hétérocycle saturé ou insaturé de quatre à huit éléments ;
la composition pharmaceutique pouvant soulager ou traiter des maladies ou des troubles physiologiques.

8. Utilisation ou composition selon la revendication 6 ou 7, dans laquelle les maladies ou les troubles physiologiques sont des maladies inflammatoires ou des maladies infectieuses.

9. Utilisation ou composition selon la revendication 6 ou 7, dans laquelle les maladies ou les troubles physiologiques sont des maladies du système immunitaire.

10. Utilisation ou composition selon la revendication 6 ou 7, dans laquelle les maladies ou les troubles physiologiques sont des tumeurs malignes.

11. Utilisation ou composition selon la revendication 6 ou 7, dans laquelle les maladies ou les troubles physiologiques sont : un choc septique, un choc endotoxique, un choc hémodynamique, un syndrome septique, une lésion de reperfusion post-ischémique, le paludisme, une infection mycobactérienne, une méningite, un psoriasis, une insuffisance cardiaque congestive, une maladie fibrotique, une cachexie, un rejet de greffe par le système immunitaire, un cancer, une maladie auto-immune, une infection opportuniste associée au SIDA, un érythème noueux lépreux, un lupus érythémateux, un lupus érythémateux réfractaire, le syndrome de Behçet, une iléite régionale, un syndrome myélodysplasique, une polyarthrite rhumatoïde (PR), une hépatite, une néphrite, une spondylarthrite ankylosante, un myélome multiple, un mélanome, une tumeur de la thyroïde, un cancer du rein, un cancer de la prostate, un lymphome, une leucémie, un cancer du foie, un gliome cérébral, un cancer colorectal, un cancer du poumon, un cancer de l'estomac, ou un cancer du sein.

12. Procédé de préparation d'un composé de formule **(I)** ou de formule **(II),** 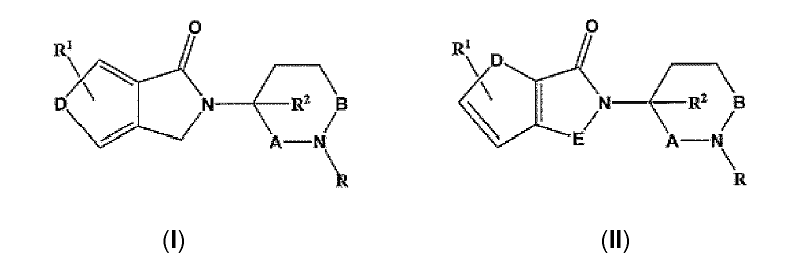 dans lesquelles
A, B et E représentent indépendamment CH₂, ou CO ;
D représente S, NH, ou N(alkyle en C₁₋₆)hydrocarbyle ;
R représente H ou R³ ;
R¹ représente H, ou un ou deux groupes identiques ou différents sont choisis parmi F, Cl, Br, (alkyle en C₁₋₄)hydrocarbyle, OH, O(alkyle en C₁₋₄)hydrocarbyle, NO₂, NHC(O)(alkyle en C₁₋₄)hydrocarbyle, NH₂, NH((alkyle en C₁₋₄)hydrocarbyle), ou N((alkyle en C₁₋₄)hydrocarbyle)₂ ;
R² dans chaque cas représente F, CF₃, H, ou (alkyle en C_{1 -4})hydrocarbyle ;
R³ dans chaque cas représente (CH₂)ₘR⁴, et m représente un nombre entier de 1 à 6 ;
R⁴ représente F, Cl, H, (alkyle en C₁₋₄)hydrocarbyle, OH, O(alkyle en C₁₋₄)hydrocarbyle, NHC(O)(alkyle en C₁₋₄)hydrocarbyle, NH₂, NH((alkyle en C₁₋₄)hydrocarbyle), N((alkyle en C₁₋₄)hydrocarbyle)₂, ou O₂CR⁵ ;
R⁵ représente CHR⁶NR⁷R⁸, CHR⁶NR⁹C(O)CHR¹⁰NR⁷R⁸, un hétérocycle W, ou CHR⁶NR⁹C(O)W ;
R⁶, R⁹ et R¹⁰ représentent indépendamment H ou (alkyle en C_{1 -4})hydrocarbyle ;
R^{7 et} R⁸ représentent indépendamment H, (alkyle en C_{1 -4})hydrocarbyle, ou pris ensemble en combinaison représentent un groupe 1,3-propylidène, 1,4-butylène, 1,5-pentylène, 1,6-hexylidène ; et
W représente un hétérocycle saturé ou insaturé de quatre à huit éléments ;
le procédé comprenant les étapes consistant à :
(1) mettre en contact un composé de formule **(VII),** ou de formule **(VIII)** avec un composé de formule **(X)** pour obtenir un composé de
formule **(XI)** ou de formule **(XII),** 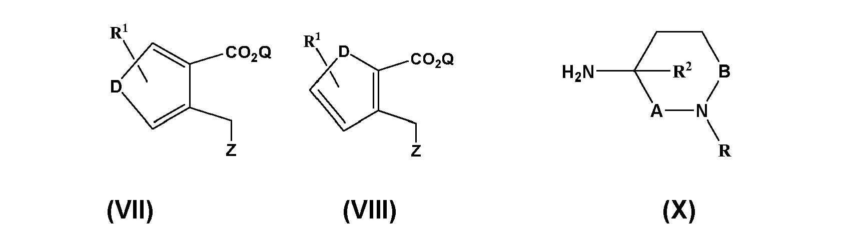 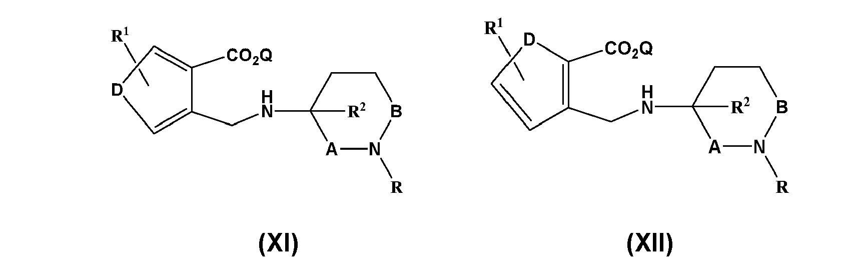 dans lesquelles les définitions de A, B, D, R, R¹, R² sont identiques à celles de la formule **(I)** ou la formule **(II),** Z représente Cl, Br, I, Ms, Ts, et Q représente un groupe méthyle ou tert-butyle ;
(2) hydrolyser le composé de formule **(XI)** ou de formule **(XII)** pour obtenir un acide correspondant de formule **(XIII)** ou de formule **(XIV)** 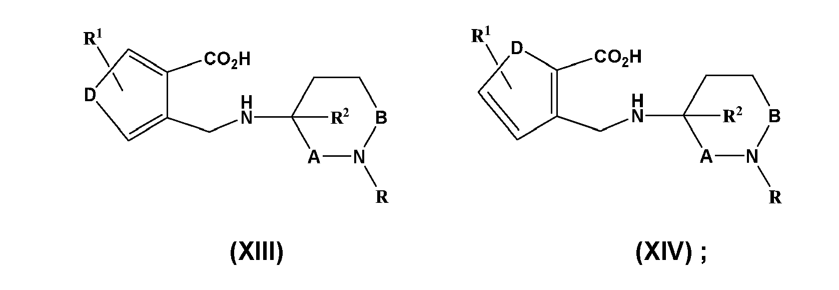 et
(3) déshydrater et cycliser le composé de formule **(XIII)** ou de formule **(XIV)** pour obtenir le composé de formule **(I)** ou de formule **(II).**

13. Procédé de préparation d'un composé de formule **(I)** ou de formule (II) comprenant la mise en contact d'un composé de formule **(XV)** ou de formule **(XVI)** avec un composé de formule L-R pour obtenir le composé de formule **(I)** ou de formule **(II),** dans lesquelles les définitions de A, B, D, E, R, R¹, R² sont identiques à celles de la formule **(I)** ou de formule **(II),** L représente Cl, Br, I, Ms ou Ts ; R est le même que pour la formule **(I)** ou la formule **(II)**

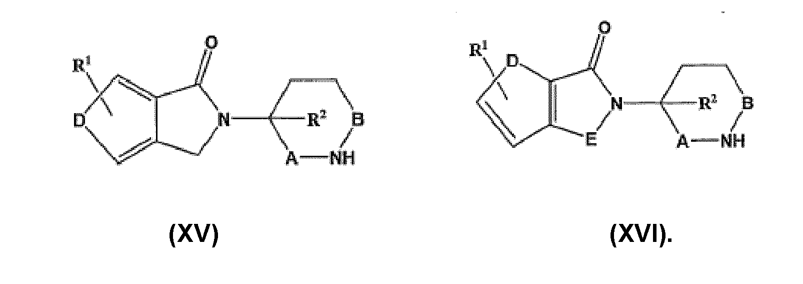

14. Composition pharmaceutique selon la revendication 7, comprenant en outre un support, un excipient, une charge, un solvant , un diluant, un agent colorant ou un adhésif pharmaceutiquement acceptable, dans laquelle le mode d'administration du composé de formule **(I)** ou de formule **(II)** est une administration par le tractus gastro-intestinal, une administration par voie orale, une injection intraveineuse, une injection intrapéritonéale, une injection sous-cutanée, une injection intramusculaire, une administration par voie intranasale, une administration par voie intraoculaire, une administration par inhalation, une administration par voie rectale, une administration par le tractus reproductif, ou une absorption percutanée.
